# EUROPEAN PATENT APPLICATION

(11) **EP 4 563 217 A1**
(43) Date of publication of application: **04.06.2025**
(21) Application number: 23846071.1
(22) Date of filing: 19.06.2023
(51) Int. Cl.: B01D 63/02, B01D 61/58, B01D 69/00, C07K 16/00, C12M 3/00, C12N 5/10, C12N 7/00, C12N 15/11, C12P 21/08

(54) **DEVICE FOR PURIFYING AND CONCENTRATING LIQUID THAT CONTAINS MINUTE USEFUL SUBSTANCE, AND METHOD FOR PRODUCING PURIFIED CONCENTRATE THAT CONTAINS MINUTE USEFUL SUBSTANCE USING SAID DEVICE**

(30) Priority: 28.07.2022 JP 2022120667
(71) Applicant: Daicel Corporation, Osaka-shi, Osaka 530-0011 (JP)
(72) Inventor: SHOJI, Koichi, Tokyo 108-8230 (JP); SATO, Hiroshi, Tokyo 108-8230 (JP); NAKATSUKA, Shuji, Tokyo 108-8230 (JP); UCHIMURA, Seiichi, Tokyo 108-8230 (JP)
(74) Representative: Grünecker Patent- und Rechtsanwälte PartG mbB
(86) International application number: PCT/JP2023/022645
(87) International publication number: WO 2024/024337

(57) **Abstract**

There are provided a purifying and concentrating apparatus for purifying and concentrating a liquid containing a minute useful substance, which can produce a liquid containing a minute useful substance with high purity and high concentration (a purified and concentrated liquid of a minute useful substance) and can more efficiently prepare the purified and concentrated liquid, in which foreign matter is less likely to be mixed from the outside in a purification and concentration process, and a method for producing a purified and concentrated liquid containing a minute useful substance using the same. A purifying and concentrating apparatus according to the present disclosure includes: a hollow fiber membrane module having a plurality of hollow fiber membranes accommodated in a container, the container having a first liquid inlet/outlet and a second liquid inlet/outlet for supplying a liquid containing a minute useful substance to the plurality of hollow fiber membranes, and a third liquid inlet/outlet for discharging the liquid having permeated through the plurality of hollow fiber membranes; a first flow path connected to the first liquid inlet/outlet and the second liquid inlet/outlet and forming a circulation path including the hollow fiber membrane module; a second flow path through which the liquid is supplied to the first flow path; and one or more pumps interposed in the first flow path and configured to circulate the liquid in a reversible manner.

## Description

### Technical Field

The present disclosure relates to a purifying and concentrating apparatus for purifying and concentrating a liquid containing a minute useful substance and a method for producing a purified and concentrated liquid containing a minute useful substance using the same.

### Background Art

As a method for separating and purifying a minute useful substance from a liquid containing the minute useful substance such as an extracellular vesicle, an antibody, a virus, a protein, or a nucleic acid, for example, a method using a separation membrane is known.

Patent Document 1 describes a method for recovering useful substances. The method includes a bleeding process of discharging a culture liquid from a cell culture tank and adding a fresh culture medium in the same amount as the discharged culture liquid to the culture tank, and a filtration process of filtering the culture liquid extracted from the culture tank using a porous membrane having substantially no dense layer. The filtration in the filtration process is tangential flow filtration, and a velocity of a permeated liquid in the filtration process is 1.0 LMH or less. Patent Document 1 also describes that the minute useful substance may be selected from the group consisting of proteins, viruses, exosomes, and nucleic acids.

Patent Document 2 describes an exosome extraction apparatus and an exosome extraction method that can efficiently collect a large amount of exosomes, which are one type of extracellular vesicles. In the exosome extraction apparatus described in Patent Document 2, a raw material liquid containing exosomes supplied from a raw material storage unit is fed to a first storage unit, and is then circulated from the first storage unit through a circulation path including a first filter and a second filter having different roles to concentrate the exosomes in the liquid.
Patent Document 1: JP 2018-76291 A
Patent Document 2: JP 2021-145649 A

### Summary of Invention

In general, an impurity is often contained in a raw material liquid containing a minute useful substance. Thus, there is a demand for an apparatus capable of preparing a liquid containing a minute useful substance with high purity and high concentration (a purified and concentrated liquid of the minute useful substance). Furthermore, there is also a demand for an apparatus capable of more efficiently preparing the purified and concentrated liquid.

In addition, a purified and concentrated liquid containing a minute useful substance may be applied to medical applications, and thus there is also a demand for an apparatus in which foreign matter is less likely to be mixed from the outside during a purification and concentration process. There is also a demand for an apparatus capable of purifying and concentrating a minute useful substance by automatic control.

The present invention has been made in view of the above circumstances, and is directed to providing a purifying and concentrating apparatus for purifying and concentrating a liquid containing a minute useful substance, which is capable of producing a liquid containing a minute useful substance with high purity and high concentration (a purified and concentrated liquid of a minute useful substance) and more efficiently preparing the purified and concentrated liquid, in which foreign matter is less likely to be mixed from the outside in a purification and concentration process, and a method for producing a purified and concentrated liquid containing a minute useful substance using the apparatus. It is also directed to providing an apparatus capable of purifying and concentrating a minute useful substance by automatic control.

To solve the problems described above, the present disclosure can adopt the following means.
[1] A purifying and concentrating apparatus for purifying and concentrating a liquid containing a minute useful substance, the purifying and concentrating apparatus including:
   a hollow fiber membrane module having a plurality of hollow fiber membranes accommodated in a container, the container having a first liquid inlet/outlet and a second liquid inlet/outlet for supplying a liquid containing a minute useful substance to the plurality of hollow fiber membranes, and a third liquid inlet/outlet for discharging the liquid having permeated through the plurality of hollow fiber membranes;
   a first flow path connected to the first liquid inlet/outlet and the second liquid inlet/outlet and forming a circulation path including the hollow fiber membrane module;
   a second flow path through which the liquid is supplied to the first flow path; and
   one or more pumps interposed in the first flow path and configured to circulate the liquid in a reversible manner.
[2] The purifying and concentrating apparatus according to [1], in which the one or more pumps each are a tube pump.
[3] The purifying and concentrating apparatus according to [1] or [2], in which a volume of the first flow path is from 5 to 100 mL.
[4] The purifying and concentrating apparatus according to any one of [1] to [3], in which the one or more pumps includes at least a first pump disposed on the first liquid inlet/outlet side and a second pump disposed on the second liquid inlet/outlet side in the first flow path.
[5] The purifying and concentrating apparatus according to any one of [1] to [4], in which each of the plurality of hollow fiber membranes has an average inner diameter of 0.2 to 1.4 mm and a molecular weight cutoff of 10000 to 1000000.
[6] The purifying and concentrating apparatus according to any one of [1] to [5], in which the first flow path is configured to be attachable and detachable.
[7] The purifying and concentrating apparatus according to any one of [1] to [6], in which the second flow path is connected to a raw material storage unit that stores a raw material liquid of the liquid containing the minute useful substance.
[8] The purifying and concentrating apparatus according to [7], in which a carrier column that removes an impurity from the liquid supplied from the raw material storage unit is interposed in the second flow path.
[9] The purifying and concentrating apparatus according to [7] or [8], in which a filtration member that removes an impurity from the liquid supplied from the raw material storage unit is interposed in the second flow path.
[10] The purifying and concentrating apparatus according to [8] or [9], further including
   a first tank that stores the liquid from which an impurity has been removed and the liquid in the first flow path,
   in which
   the first tank is connected to the second flow path, and
   the first tank is connected to a third flow path through which the liquid in the first tank is supplied toward the first flow path.
[11] The purifying and concentrating apparatus according to [10], further including a second tank that stores a diluent, the second tank being connected to a fourth flow path through which the diluent is supplied to the hollow fiber membrane module.
[12] The purifying and concentrating apparatus according to [11], in which the second tank and the first tank are connected by a fifth flow path.
[13] The purifying and concentrating apparatus according to [12], in which all of the first flow path to the fifth flow path are configured to be attachable and detachable.
[14] The purifying and concentrating apparatus according to any one of [11] to [13], further including:
   a liquid level position monitoring device including an optical sensor configured to emit light toward the third flow path and detect light transmittance; and
   a first control unit configured to control a timing at which the diluent in the second tank is supplied to the hollow fiber membrane module based on an output of the liquid level position monitoring device.
[15] The purifying and concentrating apparatus according to [14], in which the first control unit controls a timing at which the diluent in the second tank is supplied from the third liquid inlet/outlet of the hollow fiber membrane module.
[16] The purifying and concentrating apparatus according to any one of [1] to [15], further including a third tank connected to the first flow path and configured to recover the liquid in the first flow path.
[17] The purifying and concentrating apparatus according to any one of [1] to [16], in which one or more elements of the first flow path, the second flow path, and the hollow fiber membrane module are disposed in a housing.
[18] The purifying and concentrating apparatus according to [17], further including
   a management unit configured to manage a degree of cleanliness in the housing, in which
   the management unit includes a fan filter device that supplies filtered air.
[19] The purifying and concentrating apparatus according to [18], in which the management unit includes a UV lamp.
[20] The purifying and concentrating apparatus according to any one of [1] to [19], in which the minute useful substance is selected from the group consisting of an extracellular vesicle, an antibody, a virus, a protein, an enzyme, and a nucleic acid.
[21] The purifying and concentrating apparatus according to any one of [1] to [20], in which at least the hollow fiber membrane module and the first flow path are accommodated in a case of an attachable and detachable cartridge.
[22] The purifying and concentrating apparatus according to any one of [8] to [21], in which at least the second flow path and the carrier column are accommodated in a case of an attachable and detachable cartridge.
[23] The purifying and concentrating apparatus according to [22], in which the hollow fiber membrane module and the first flow path are accommodated in the case.
[24] The purifying and concentrating apparatus according to any one of [15] to [23], in which the hollow fiber membrane has at least two third liquid inlets/outlets, and the first control unit supplies the diluent from one of the at least two third liquid inlets/outlets in a state where flow of the liquid through the first liquid inlet/outlet and the second liquid inlet/outlet of the hollow fiber membrane module is stopped, and then supplies the diluent from one of the at least two third liquid inlets/outlets in a state where the flow of the liquid through the first liquid inlet/outlet and the second liquid inlet/outlet of the hollow fiber membrane module is enabled.
[25] A method for producing a purified and concentrated liquid containing a minute useful substance using the purifying and concentrating apparatus described in any one of [1] to [24], the method including:
   supplying a raw material liquid of a liquid containing a minute useful substance to the purifying and concentrating apparatus; and
   purifying and concentrating the liquid with the hollow fiber membrane module,
   in which
   the purifying and concentrating the liquid is performed while circulating the liquid in the circulation path including the hollow fiber membrane module in a reversible manner.
[26] The method for producing a purified and concentrated liquid according to [25], the method further including changing a circulation direction of the liquid in the first flow path depending on a purifying and concentrating time of the liquid or depending on a flow rate of the liquid circulated in the circulation path.
[27] The method for producing a purified and concentrated liquid according to [25] or [26], in which the liquid is supplied to the hollow fiber membrane module to be purified and concentrated in such a manner that a hollow fiber transmembrane pressure in the hollow fiber membrane module is from 0.02 to 0.2 MPa.
[28] The method for producing a purified and concentrated liquid according to [27], the method further including feeding the liquid in the first flow path toward the hollow fiber membrane module by
   setting a liquid feeding direction of a first pump disposed on the first liquid inlet/outlet side, and a liquid feeding direction of a second pump disposed on the second liquid inlet/outlet side to be clockwise or counterclockwise, the first pump and the second pump being among two or more of the one or more pumps interposed in the first flow path, and
   setting an output of one of the first pump and the second pump as a main pump to be higher than an output of the other pump.
[29] The method for producing a purified and concentrated liquid according to any one of [25] to [28], in which the purifying and concentrating the liquid with the hollow fiber membrane module includes subjecting the liquid to alternating tangential flow filtration with the hollow fiber membrane module.
[30] The method for producing a purified and concentrated liquid according to any one of [25] to [29], in which the purifying and concentrating the liquid with the hollow fiber membrane module includes subjecting the liquid to alternating tangential flow filtration with the hollow fiber membrane module while recovering the minute useful substance deposited on membrane surfaces of the hollow fiber membrane.
[31] The method for producing a purified and concentrated liquid according to any one of [25] to [30], in which
   the purifying and concentrating the liquid with the hollow fiber membrane module includes diluting the concentrated liquid with a diluent by from 2 to 200 times, and then further purifying and concentrating the diluted liquid, and
   a timing of supplying a diluent to the concentrated liquid is determined by a liquid level position of the liquid in the first flow path.
[32] The method for producing a purified and concentrated liquid according to any one of [25] to [31], the method further including recovering an entire amount of the purified and concentrated liquid in the first flow path.
[33] The method for producing a purified and concentrated liquid according to any one of [25] to [32], in which the minute useful substance is selected from the group consisting of an extracellular vesicle, an antibody, a virus, a protein, an enzyme, and a nucleic acid.
[34] The method for producing a purified and concentrated liquid according to any one of [25] to [33], in which the purifying and concentrating the liquid with the hollow fiber membrane module includes subjecting the hollow fiber membrane module to flushing treatment.

According to the present disclosure, it is possible to provide a purifying and concentrating apparatus for purifying and concentrating a liquid containing a minute useful substance, which can produce a liquid containing a minute useful substance with high purity and high concentration (a purified and concentrated liquid of a minute useful substance) and can more efficiently prepare the purified and concentrated liquid, in which foreign matter is less likely to be mixed from the outside in a purification and concentration process, and a method for producing a purified and concentrated liquid containing a minute useful substance using the same. The purifying and concentrating apparatus according to an embodiment of the present disclosure is also useful as an apparatus capable of purifying and concentrating a minute useful substance by automatic control.

### Brief Description of Drawings

FIG. 1 is a schematic configuration diagram illustrating an example of a purifying and concentrating apparatus according to the present disclosure.
FIG. 2 is a schematic cross-sectional view illustrating an example of a hollow fiber membrane module in the purifying and concentrating apparatus according to the present disclosure.
FIG. 3 is a schematic configuration diagram illustrating an example of the purifying and concentrating apparatus according to the present disclosure.
FIG. 4 is a schematic configuration diagram illustrating an example of the purifying and concentrating apparatus according to the present disclosure.
FIG. 5 is a schematic configuration diagram illustrating an example of the purifying and concentrating apparatus according to the present disclosure.
FIG. 6 is a schematic configuration diagram illustrating an example of the purifying and concentrating apparatus according to the present disclosure.
FIG. 7 is a schematic configuration diagram illustrating an example of the purifying and concentrating apparatus according to the present disclosure.
FIG. 8 is a schematic configuration diagram illustrating an example of the purifying and concentrating apparatus according to the present disclosure.
FIG. 9 is a schematic configuration diagram illustrating an example of the purifying and concentrating apparatus according to the present disclosure.
FIG. 10 is a flowchart illustrating an example of purifying and concentrating treatment using the purifying and concentrating apparatus according to an embodiment of the present disclosure.
FIG. 11 is a schematic configuration diagram illustrating an example of the purifying and concentrating apparatus according to the present disclosure.
FIG. 12 is a schematic configuration diagram illustrating an example of the purifying and concentrating apparatus according to the present disclosure.
FIG. 13 is a schematic configuration diagram illustrating an example of the purifying and concentrating apparatus according to the present disclosure.
FIG. 14 is a graph showing results of measuring concentrated liquids prepared in Example 2 and Comparative Example 2 by BCA assay.
FIG. 15 is an absorbance chart of the concentrated liquids prepared in Example 2 and Comparative Example 2.

### Description of Embodiments

Hereinafter, an example of the present disclosure will be explained with reference to the drawings. However, each of the configurations, combinations thereof, and the like in each embodiment are an example, and various additions, omissions, substitutions, and other changes may be made as appropriate without departing from the spirit of the present disclosure. The present disclosure is not limited by the embodiments and is limited only by the claims. In addition, in a case where a plurality of upper limit values and lower limit values are described for a specific parameter, any upper limit value and any lower limit value among these upper limit values and lower limit values can be combined to obtain a suitable numerical range.

In the present disclosure, a liquid that has permeated through the hollow fiber membrane in a hollow fiber membrane module is referred to as a "permeated liquid", and a liquid that has not permeated through the hollow fiber membrane and remains in the hollow fiber membrane is referred to as a "concentrated liquid". Note that the "concentrated liquid" refers to any liquid purified and concentrated by a hollow fiber membrane.

### Purifying and Concentrating Apparatus of Liquid Containing Minute Useful Substance

A purifying and concentrating apparatus for purifying and concentrating a liquid containing a minute useful substance according to an embodiment of the present disclosure includes:
a hollow fiber membrane module having a plurality of hollow fiber membranes accommodated in a container, the container having a first liquid inlet/outlet and a second liquid inlet/outlet for supplying a liquid containing a minute useful substance to the plurality of hollow fiber membranes, and a third liquid inlet/outlet for discharging the liquid having permeated through the plurality of hollow fiber membranes;
a first flow path connected to the first liquid inlet/outlet and the second liquid inlet/outlet and forming a circulation path including the hollow fiber membrane module;
a second flow path through which the liquid is supplied to the first flow path; and
one or more pumps interposed in the first flow path and configured to circulate the liquid in a reversible manner. Hereinafter, a first embodiment of the purifying and concentrating apparatus of the present disclosure will be described with reference to the drawings.

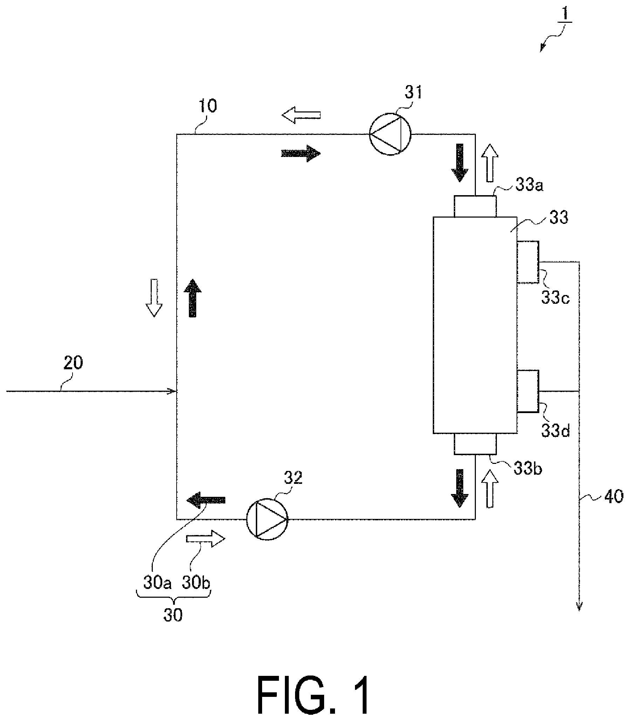

FIG. 1 is a schematic configuration diagram illustrating an example of a purifying and concentrating apparatus 1 (hereinafter, referred to as the "apparatus 1") according to the present disclosure. The apparatus 1 in FIG. 1 includes a hollow fiber membrane module 33 having a plurality of hollow fiber membranes accommodated in a container, the container including a first liquid inlet/outlet 33a and a second liquid inlet/outlet 33b for supplying a liquid containing a minute useful substance to the plurality of hollow fiber membranes and third liquid inlets/outlets 33c and 33d for discharging the liquid having permeated through the plurality of hollow fiber membranes; a first flow path 10 connected to the first liquid inlet/outlet 33a and the second liquid inlet/outlet 33b and forming a circulation path 30 including the hollow fiber membrane module 33; a second flow path 20 through which the liquid is supplied to the first flow path 10; and one or more pumps interposed in the first flow path 10 and configured to circulate the liquid in a reversible manner. Note that in the apparatus 1 in FIG. 1, a first pump 31 and a second pump 32 are interposed in the first flow path 10. According to this configuration, a liquid containing a minute useful substance can be purified and concentrated while being circulated in the first flow path 10 connected to the hollow fiber membrane module 33 in a reversible manner. This makes it possible to efficiently produce a purified and concentrated liquid of a minute useful substance. In addition, a container for performing a purifying and concentrating treatment is not required, and thus the apparatus can be made compact. As a result, the apparatus 1 can be adjusted to a size that can be disposed in a clean booth or the like, and foreign matter can be prevented from being mixed from the outside during the purifying and concentrating treatment.

### First flow path

In the apparatus 1 in FIG. 1, the first flow path 10 is connected to the hollow fiber membrane module 33 to form the circulation path 30. One or more pumps are interposed in the first flow path 10. Note that in the present disclosure, a "flow path" refers to a conduit through which a liquid flows. In addition, a "circulation path" is a path configured in such a manner that a liquid in a flow path makes one round and returns to the original position, and a circulation direction is indicated by an arrow in the drawings.

In the apparatus 1 in FIG. 1, the first flow path 10 is attachable to and detachable from one or more pumps and the hollow fiber membrane module 33. In a case of the apparatus 1 in FIG. 1, the first flow path 10 may be formed of a resin tube, and the resin tube may be connected to one or more pumps and the hollow fiber membrane module 33. When the first flow path 10 is configured to be attachable to and detachable from other elements as described above, the first flow path 10 can be removed from other elements and discarded after the purifying and concentrating treatment of the liquid containing the minute useful substance is performed. According to this configuration, the first flow path 10 can be discarded and replaced with a new flow path for each batch, which makes foreign matter less likely to be mixed. In addition, the apparatus 1 can be made more compact. Furthermore, when a flow path is formed of a resin tube, the apparatus 1 can be produced at a low cost. The resin tube forming the first flow path 10 is not particularly limited, and from the viewpoint of excellent toughness, chemical resistance, recovery elasticity, cleanness, and the like, a thermoplastic elastomer tube (for example, a PharMed tube and the like), an ester-based polyurethane tube (for example, a TYGON tube and the like), a nylon tube, a silicon tube, a polyethylene tube, and the like are preferable.

In the first flow path 10 illustrated in FIG. 1, a circulation path 30a through which the liquid flows clockwise and a circulation path 30b through which the liquid flows counterclockwise are formed. In the apparatus 1 in FIG. 1, the liquid supplied from the second flow path 20 into the first flow path 10 is fed to circulate through the circulation path 30a or the circulation path 30b by the first pump 31 and the second pump 32. The liquid in the first flow path 10 is purified and concentrated with the hollow fiber membrane module 33 while circulating through the circulation path 30a or the circulation path 30b. Note that the circulation path 30a and the circulation path 30b are collectively referred to as the "circulation path 30 ". The liquid having permeated through the hollow fiber membranes in the hollow fiber membrane module 33 is discharged from the third liquid inlets/outlets 33c and 33d to a waste liquid tank (to be described below) via a flow path 40.

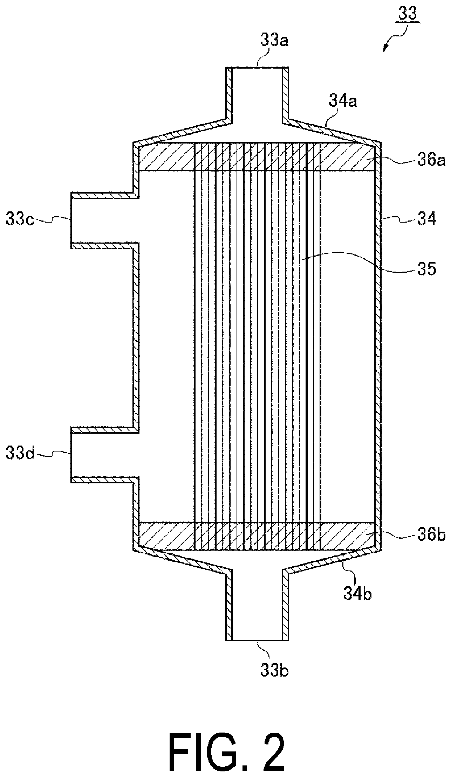

In the apparatus 1 illustrated in FIG. 1, the first flow path 10 is connected to the first liquid inlet/outlet 33a and the second liquid inlet/outlet 33b of the hollow fiber membrane module 33. In the hollow fiber membrane module 33, as illustrated in FIG. 2, a hollow fiber membrane bundle 35 is arranged in parallel with the longitudinal direction of the hollow fiber membrane module 33. Thus, the liquid having entered from the first liquid inlet/outlet 33a of the hollow fiber membrane module 33 is purified and concentrated by tangential flow filtration in which the liquid flows parallel to membrane surfaces in the longitudinal direction of the hollow fiber membrane bundle 35, and then discharged from the second liquid inlet/outlet 33b. Similarly, the liquid having entered from the second liquid inlet/outlet 33b is also subjected to tangential flow filtration and discharged from the first liquid inlet/outlet 33a. That is, in the apparatus 1 in FIG. 1, the liquid can be purified and concentrated while being subjected to alternating tangential flow filtration. When these operations are alternately repeated, the minute useful substance and the impurity are hardly deposited on the surface of the hollow fiber membrane. As a result, it is possible to suppress a decrease in liquid permeation rate and to maintain high treatment efficiency. That is, it is possible to more efficiently produce the liquid containing the minute useful substance with higher purity and higher concentration.

In the apparatus 1 in FIG. 1, a volume of the first flow path 10 is preferably from 5 to 100 mL, and more preferably from 8 to 50 mL. Note that the volume of the first flow path 10 refers to a volume of the entire flow path. For example, in a case where the first flow path 10 is formed of a resin tube, the volume of the first flow path 10 can be adjusted by changing a length or an inner diameter of the tube. Note that the volume (liquid amount) of the liquid in the first flow path 10 may be determined on the basis of information of a liquid level position monitoring device 11 described below.

### Pump

The one or more pumps interposed in the first flow path 10 are preferably tube pumps. In the apparatus 1 in FIG. 1, the two pumps in the first flow path 10 are tube pumps. In a case where the first flow path 10 is formed of a resin tube, when the one or more pumps in the first flow path 10 are tube pumps, the resin tube can be attached and detached more easily. Further, the apparatus can be made more compact. In addition, it is possible to make the apparatus 1 have a simpler (simpler and less expensive) configuration.

In the apparatus 1 in FIG. 1, the first pump 31 is disposed on the first liquid inlet/outlet 33a side of the hollow fiber membrane module 33, and the second pump 32 is disposed on the second liquid inlet/outlet 33b side. When two or more, preferably two pumps are arranged to sandwich the hollow fiber membrane module 33, the liquid can be circulated more efficiently.

### Hollow fiber membrane module

The apparatus 1 in the present disclosure includes the hollow fiber membrane module 33 having a plurality of hollow fiber membranes accommodated in a container having a liquid inlet/outlet.

### Hollow fiber membrane

The material of the hollow fiber membrane of the present disclosure can be appropriately designed depending on the purpose of use of the present disclosure, the properties of the liquid containing the minute useful substance, and the like.

Specifically, an organic polymer-based material such as polyvinylidene fluoride (PVDF), polyether sulfone (PES), polysulfone (PSf), polyolefin (PE, PP, PTFE, or the like), polyamide, polyacrylonitrile, or cellulose acetate (CA), or a modified product thereof can be used.

In addition, a composite hollow fiber membrane including a plurality of components selected from the above can also be used.

Among them, in a case where the minute useful substance is a biomolecule such as an exosome, it is preferable to use a hollow fiber membrane made of cellulose acetate (CA). When the hollow fiber membrane made of CA (hereinafter, sometimes referred to as a "CA membrane") is used, it is possible to suppress a decrease in filtration rate during the purifying and concentrating treatment and to further improve the effect of recovering the minute useful substance deposited on a membrane surface of the hollow fiber membrane. The reason for this is as follows. The surface charge of the biomolecule is a weak negative charge and the CA membrane also has a weak negative charge. Thus, the biomolecule and the CA membrane are not attracted to each other by a strong Coulomb force, and the shear force of the cross-flow filtration easily functions by appropriately controlling a circulation linear velocity. In addition, when the CA membrane is used, it is possible to more efficiently recover the minute useful substance deposited on the membrane surface of the hollow fiber membrane by a method described below.

The material of the hollow fiber membrane of the present disclosure can be appropriately designed depending on the purpose of use of the present disclosure, the properties of the raw material liquid containing the minute useful substance, and the like. The hollow fiber membrane can also be coated with an appropriate substance to improve fouling resistance of the surface of the hollow fiber membrane, depending on the properties of the raw material liquid containing the minute useful substance.

The coating treatment can be performed, for example, by the same method as the method of bonding a cationic polymer to the surface of a hollow fiber membrane made of sulfonated polyether sulfone and polyether sulfone described in Japanese Patent No. 6081290.

That is, there can be used a method in which the hollow fiber membrane is immersed in an aqueous solution (aqueous dispersion) of a coating substance, or a method in which an aqueous solution (aqueous dispersion) of a coating substance is caused to pass through the hollow fiber membrane in a state of the hollow fiber membrane or hollow fiber membrane module to adsorb and bond the coating substance to the surface of the hollow fiber membrane, and then the coating substance just deposited on the thin layer of the coating substance is removed by washing with water.

In a case of concentrating and purifying a certain type of extracellular vesicle in a certain type of culture supernatant, it may be preferable to coat a hollow fiber membrane made of cellulose acetate with a certain type of biomolecule, in terms of purifying and concentrating efficiency and improvement in recovery rate of the minute useful substance such as an extracellular vesicle.

As an example, a cellulose acetate hollow fiber membrane module (molecular weight cutoff of 300000, membrane area of 0.062 m²) is subjected to coating treatment in which 1.3 mg/mL of fat-free milk powder solution prepared by dissolving fat-free milk powder in phosphate buffer solution (PBS) is caused to pass therethrough at a liquid flow rate of 200 mL/min for 10 minutes to adsorb and bind biomolecules such as casein in the fat-free milk powder to the surface of the hollow fiber membrane as a thin layer. Then, the hollow fiber membrane module is washed with 250 mL of phosphate buffer solution (PBS) to remove excess components just deposited on the thin layer. The resulting hollow fiber membrane module can be used.

In one embodiment, from the viewpoint of enhancing the fouling resistance of the hollow fiber membrane, a biomolecule of a type different from the minute useful substance is preferably attached to at least a partial region of the hollow fiber membrane. The biomolecule is more preferably attached to a half or more region of the surface of the hollow fiber membrane, and is preferably attached to the entire region. In one embodiment, a layer containing a biomolecule of a type different from the minute useful substance is preferably formed on at least a part of the surface of the hollow fiber membrane. The "layer containing a biomolecule" means that a substance containing a biomolecule is attached to at least a partial region of the hollow fiber membrane with a certain breadth. The layer containing a biomolecule is preferably formed in a half or more region of the surface of the hollow fiber membrane, and more preferably formed in the entire region. In one embodiment, at least a part of the surface of the hollow fiber membrane is preferably coated with a biomolecule of a type different from the minute useful substance.

The biomolecule of a type different from the minute useful substance is prepared from an animal cell tissue such as blood, milk, or cartilage, or another biological cell tissue. Typical examples thereof include biomolecules contained in skim milk, fat-free milk powder, and the like (e.g., casein); biomolecules contained in serum and the like (e.g., serum globulin, serum albumin); and gelatin (e.g., fish gelatin), and may be one or more selected from these.

### Average inner diameter of hollow fiber membrane

The average inner diameter of the hollow fiber membrane of the present disclosure can be appropriately designed depending on the purpose of use of the present disclosure, the properties of the liquid containing the minute useful substance, and the like. In one embodiment, a hollow fiber membrane having an average inner diameter of from 0.2 mm to 1.4 mm can be employed. Note that the "average inner diameter of the hollow fiber membrane" in the present disclosure is a value determined by cutting one hollow fiber membrane along any plane perpendicular to the longitudinal direction and calculating from the diameter of the smallest circle inscribed in the hollow portion of the cut surface. In the present disclosure, the diameter is measured for any 10 or more and 100 or less cut surfaces, and an average value of the measured values is defined as the "average inner diameter".

Note that the hollow fiber membrane of the present disclosure may have an average outer diameter of from 0.3 mm to 2.0 mm. In one embodiment, in a case where the average inner diameter of the hollow fiber membrane is from 0.2 mm to 1.0 mm, the average outer diameter may be from 0.3 mm to 1.4 mm. In another embodiment, in a case where the average inner diameter of the hollow fiber membrane is from 1.0 mm to 1.4 mm, the average outer diameter may be from 1.5 mm to 2.0 mm. Note that the "average outer diameter of the hollow fiber membrane" is a value calculated from the diameter of the smallest circle surrounding the outer edge of the cut surface of the hollow fiber membrane cut by the same method as the above-described average inner diameter of the hollow fiber membrane. In the present disclosure, the diameter is measured for any 10 or more and 100 or less cut surfaces, and an average value of the measured values is defined as the "average outer diameter".

In the purpose of use of the present disclosure, the average inner diameter of the hollow fiber membrane may be from 0.6 mm to 1.4 mm from the viewpoint of easily ensuring a thickness at which the hollow fiber membrane does not become excessively flexible and easily improving the recovery efficiency of the minute useful substance deposited on the membrane surface of the hollow fiber membrane.

Further, the average inner diameter may be adjusted by a liquid viscosity of the liquid containing the minute useful substance during operation. In a case where the liquid viscosity is high (for example, in a case where the liquid viscosity at a liquid temperature of 20°C and a shear rate of 25 s⁻¹ is from 10 to 30 mPa·s), it is preferable to use a large-diameter hollow fiber membrane having an average inner diameter of 1.0 mm to 1.4 mm. In a case where the liquid viscosity of the liquid containing the minute useful substance during operation is low (for example, in a case where the liquid viscosity at a liquid temperature of 20°C and a shear rate of 25 s⁻¹ is less than 10 mPa·s), it is preferable to use a hollow fiber membrane having an average inner diameter of 0.6 mm to 1.0 mm. Note that when the average inner diameter of the hollow fiber membranes increases, an effective membrane area tends to decrease in a case of using the hollow fiber membranes as a hollow fiber membrane module. Accordingly, it is preferable to use hollow fiber membranes having an average inner diameter of from 0.2 mm to 1.0 mm from the viewpoint of easily suppressing a decrease in effective membrane area in a case of being formed into the hollow fiber membrane module.

### Molecular weight cutoff of hollow fiber membrane

The molecular weight cutoff of the hollow fiber membrane of the present disclosure can be appropriately designed depending on the purpose of use of the present disclosure, the properties of the raw material liquid containing the minute useful substance, and the like. In one embodiment, the molecular weight cutoff may be from 5000 to 3000000, or from 10000 to 1000000. In a case where the minute useful substance is a specific protein, it is preferable to use a hollow fiber membrane having a molecular weight cutoff slightly lower than the molecular weight of the protein (for example, a hollow fiber membrane having a molecular weight cutoff about 10 to 50% lower than the molecular weight of the protein). In one embodiment, in a case where the minute useful substance is an exosome, the molecular weight cutoff of the hollow fiber membrane is preferably from 50000 to 1000000, more preferably from 70000 to 800000, and even more preferably from 100000 to 500000.

In a case where the minute useful substance is an extracellular vesicle, a γ-globulin permeability of the hollow fiber membrane module of the present disclosure is preferably from 5 to 90%, and more preferably from 10 to 80%, from the viewpoint of efficiency of purifying and concentrating. In the present disclosure, a method for measuring a permeability of an impurity in the liquid containing the minute useful substance, including the above-described γ-globulin, is not particularly limited, and a method known in the related art can be employed. Specifically, it is possible to employ a method of measuring a permeability of a standard substance having a molecular structure similar to that of the impurity and a molecular weight similar to that of the impurity. In a case of measuring the permeability, the hollow fiber membrane module may be used, or one hollow fiber membrane used in the hollow fiber membrane module may be directly used. Furthermore, the permeability of an internal pressure type hollow fiber membrane module can be substituted by a permeability measured by a method in which in one hollow fiber membrane, pressure is applied from the outside of the hollow fiber membrane to allow permeation to the inside.

The number of hollow fiber membranes used in the hollow fiber membrane module of the present disclosure is not particularly limited. Depending on the purpose of use of the present disclosure, a weight of the minute useful substance to be purified and concentrated, and the like, the number of hollow fiber membranes to be incorporated into the module can be appropriately designed to provide a necessary effective membrane area.

The hollow fiber membranes are preferably bundled with an adhesive or the like and accommodated in the container. In the present disclosure, when the liquid containing the minute useful substance is supplied from the first liquid inlet/outlet and/or the second liquid inlet/outlet of the hollow fiber membrane module, the liquid flows to one side of the hollow fiber membrane bundle (inside or outside of the hollow fiber membrane bundle) in the container, and is purified and concentrated by the hollow fiber membrane bundle. The permeated liquid that has permeated through the hollow fiber membrane bundle flows out to the other side of the hollow fiber membrane bundle (outside or inside of the hollow fiber membrane bundle) and is discharged from the third liquid inlets/outlets. At this time, the concentrated liquid remaining in the inside or outside of the hollow fiber membrane bundle without permeating can be discharged (recovered) from the first liquid inlet/outlet and/or the second liquid inlet/outlet to the outside of the hollow fiber membrane module.

A hollow fiber membrane module in which the liquid containing the minute useful substance is supplied to the inside of the hollow fiber membrane bundle is an internal pressure type hollow fiber membrane module. Hereinafter, an example of the internal pressure type hollow fiber membrane module will be described as an embodiment of the hollow fiber membrane module in the present disclosure.

FIG. 2 is a schematic cross-sectional view illustrating an example of an internal pressure type hollow fiber membrane module 33. In the hollow fiber membrane module 33, a hollow fiber membrane bundle 35 in which a plurality of hollow fiber membranes are bundled is accommodated in a cylindrical container 34. The plurality of hollow fiber membranes are bundled with a biocompatible sealing adhesive (for example, a polyurethane-based sealing adhesive or the like) to form the hollow fiber membrane bundle 35, and are bonded and fixed to both ends of the inside of the cylindrical container 34 in a longitudinal direction thereof with adhesive layers 36a and 36b made of the sealing adhesive. From the viewpoint of transparency, the cylindrical container 34 may be made of a material such as polycarbonate, poly(meth)acrylate, or polysulfone. Note that the plurality of hollow fiber membranes are open at both end surfaces 34a and 34b of the cylindrical container 34. The first liquid inlet/outlet 33a and the second liquid inlet/outlet 33b are provided at both ends of the hollow fiber membrane module 33 in the longitudinal direction. In addition, the third liquid inlets/outlets 33c and 33d are provided in a side surface portion of the hollow fiber membrane module 33 in the longitudinal direction. The liquid supplied from the first liquid inlet/outlet 33a and/or the second liquid inlet/outlet 33b is fed to the inside of the hollow fiber membrane bundle 35 to be purified and concentrated. The permeated liquid that has permeated to the outside of the hollow fiber membrane bundle 35 is discharged from the third liquid inlets/outlets 33c and 33d provided on the side surface of the cylindrical container 34. Note that the third liquid inlets/outlets are preferably provided at least two positions, that is, an upper portion and a lower portion of the side surface of the cylindrical container 34. In FIG. 2, the third liquid inlets/outlets 33c and 33d are provided only on one side surface portion of the hollow fiber membrane module 33, but the present disclosure is not limited thereto. The third liquid inlets/outlets 33c and 33d may be provided separately on one side surface portion and the other side surface portion.

### Filling rate in hollow fiber membrane module

A filling rate in the hollow fiber membrane module of the present disclosure is preferably from 5 to 55%, more preferably from 20 to 55%, and still more preferably from 25 to 55%. The filling rate refers to a percentage of the total sum of cross-sectional areas of outer diameter portions of the hollow fiber membrane bundle in the cross-sectional area of the hollow fiber membrane module, generally in the transverse direction of a cylindrical container. When the filling rate is from 5 to 45%, the minute useful substance deposited on the membrane surfaces of the hollow fiber membrane can be more efficiently recovered while optimizing the size of the hollow fiber membrane module.

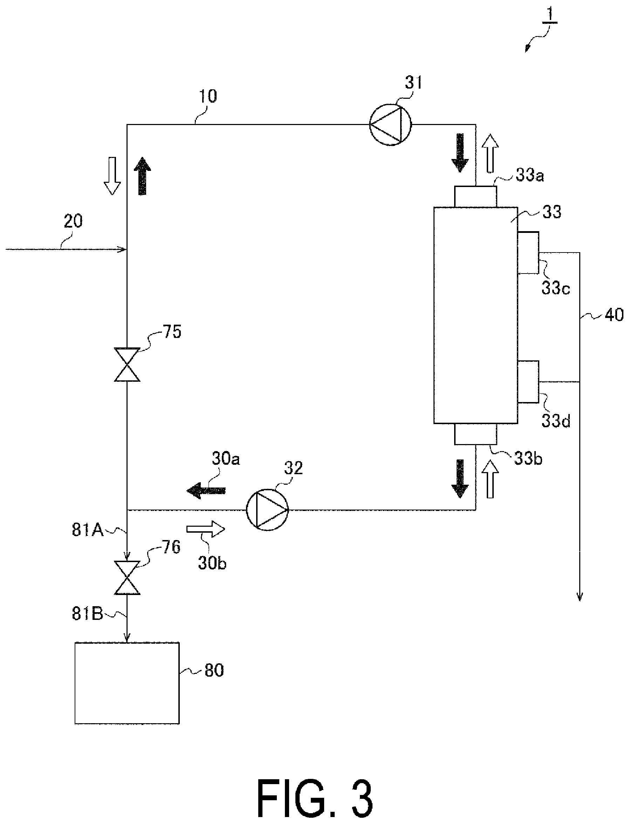

The apparatus 1 in FIG. 3 includes a third tank 80 that is connected to the first flow path 10 and recovers the liquid in the first flow path 10. The third tank 80 is connected to a flow path 81B, and the flow path 81B is connected to a flow path 81A via an on-off valve 76. The flow path 81A is further connected to the first flow path 10. An on-off valve 75 is also provided in the first flow path 10. While the liquid is being purified and concentrated, the on-off valve 75 is opened and the on-off valve 76 is closed. When the purifying and concentrating treatment is completed, the on-off valve 75 is closed and the on-off valve 76 is opened. As a result, the entire amount of the concentrated liquid in the first flow path 10 is recovered into the third tank 80.

The recovery of the concentrated liquid into the third tank 80 can be controlled by a second control unit.

In the apparatus 1 in FIG. 3, the second control unit can first open the on-off valve 76 to feed clean gas from an air bleeding portion using the first pump 31 and the second pump 32. Further, the concentrated liquid in the first flow path 10 can be recovered into the third tank 80 by closing the on-off valve 75 at a freely-selected timing while feeding the air. Furthermore, the second control unit can operate the first pump 31 and the second pump 32 in such a manner that the concentrated liquid in the first flow path 10 circulates through the circulation path 30a. That is, the first pump 31 and the second pump 32 can be operated in such a manner that their liquid feeding directions are the same clockwise direction. With the on-off valve 75 closed, the concentrated liquid flowing through the circulation path 30a can be fed to the third tank 80 via the flow paths 81A and 81B. In addition, the second control unit can start recovery treatment after the liquid level position monitoring device 11 described below determines that concentrating treatment in first flow path 10 is completed. Alternatively, the operation of recovering the concentrated liquid in the first flow path 10 can be manually operated by a person.

### Second Flow Path

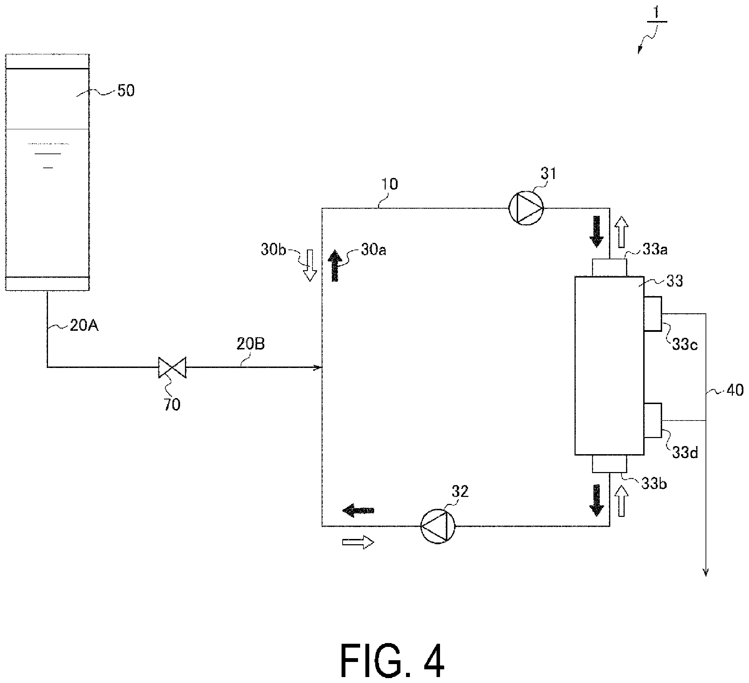

The second flow path 20 supplies the liquid containing the minute useful substance to the first flow path 10. FIGS. 4 to 8 illustrate some typical schematic configurations of the second flow path 20. Note that the purifying and concentrating apparatus 1 of the present disclosure may include a valve, an automatic valve, a backflow prevention valve, an orifice, a flowmeter, a pressure gauge, a liquid level gauge, a safety device such as a rupture disk, and the like, which are omitted in the schematic configuration diagram. Note that sensor portions of the flowmeter and the pressure gauge preferably come into contact with the liquid containing the minute useful substance through a tube (i.e., the sensor portions do not come into direct contact with the liquid), from the viewpoint of easy attachment and detachment. As the flowmeter, for example, a clamp-on integrating flowmeter or the like can be preferably used.

In the apparatus 1 in FIG. 4, the second flow path 20 is connected to a raw material storage unit 50 that stores a raw material liquid of the liquid containing the minute useful substance. In the apparatus 1 in FIG. 4, the second flow path 20 is divided into flow paths 20A and 20B with an on-off valve 70 interposed therebetween, the flow path 20A is connected to the raw material storage unit 50, and the flow path 20B is connected to the first flow path 10. In the apparatus 1 in FIG. 4, with the on-off valve 70 opened, the raw material liquid in the raw material storage unit 50 can be supplied to the first flow path 10. Note that the direction of gravity is not reflected in the drawings of the present disclosure.

### Raw material storage unit

The raw material storage unit 50 includes a container that stores the raw material liquid. The container may be fixed to the apparatus 1, or may be installed to be easily detachable. In a case where the container is installed to be easily detachable, the raw material storage unit 50 can include various types of equipment such as a fixing member for fixing the container.

A shape and a volume of the container that stores the raw material liquid are not particularly limited, and can be determined depending on a type of the minute useful substance, an amount of the raw material liquid, or the like. In one embodiment, in a case of purifying and concentrating a raw material liquid containing a minute useful substance selected from an extracellular vesicle, an antibody, a virus, a protein, an enzyme, a nucleic acid, or the like, the volume of the container may be 50 to 5000 mL or 200 to 2000 mL. The container may be formed of an infusion pack for easy batch-by-batch removal.

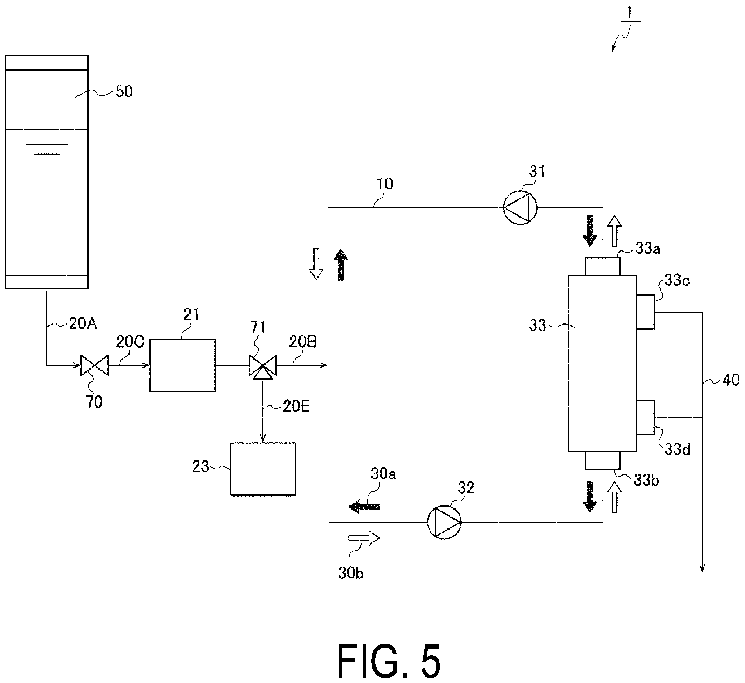

In the apparatus 1 in FIG. 5, the carrier column 21 that removes an impurity from the raw material liquid supplied from the raw material storage unit 50 is interposed in the second flow path. In the apparatus 1 in FIG. 5, the second flow path 20 is divided into the flow path 20A connected to the raw material storage unit 50, a flow path 20C for supplying the raw material liquid to the carrier column 21, and the flow path 20B for supplying the liquid treated in the carrier column 21 (hereinafter, referred to as a "roughly purified liquid") to the first flow path 10, and the on-off valve 70 is disposed between the flow path 20A and the flow path 20C. With the on-off valve 70 opened, the raw material liquid in the raw material storage unit 50 is supplied to the carrier column 21. In addition, a three-way valve 71 is interposed in the flow path 20B, and a flow path 20E through which the liquid flows toward a waste liquid tank 23 is connected to one end of the three-way valve 71.

### Carrier column

The carrier column 21 is filled with a material capable of removing an impurity other than the minute useful substance from the raw material liquid. The material is preferably an adsorbent capable of adsorbing and removing an impurity from the raw material liquid.

The adsorbent is preferably a porous granular material capable of adsorbing and holding the impurity. The porous granular material refers to a granular material having a large number of pores on the surface and inside thereof, and a porous granular material of a polymer or a porous granular material of an inorganic material can be used. In a case where the minute useful substance contained in the raw material liquid to be treated by the apparatus 1 is a biological substance such as an extracellular vesicle, an antibody, a virus, a protein, an enzyme, or a nucleic acid, the porous granular material is preferably a material in which insides of pores are positively charged.

For these porous granular materials, two or more porous granular materials having one or different adsorption performances may be used in combination.

Examples of the two or more porous granular materials having different adsorption performances include porous granular materials having different pore sizes, porous granular materials having different specific surface areas, porous granular materials having different average particle sizes, porous granular materials having different types of substrates constituting the porous granular materials, and porous granular materials having different types and densities of functional groups present on inner surfaces of the pores.

Examples of the substrate of such a porous granular material include polysaccharides such as cellulose, agarose, starch, amylose, dextran, pullulan, and glucomannan; polyacrylic acids or derivatives thereof; synthetic polymers such as polyvinyl alcohol, nylon, polysulfone, polyacrylonitrile, polyethylene, polypropylene, and polystyrene; glass; porous glass; silica gel; and hydroxyapatite. One type of these may be used alone, or two or more types thereof may be used in combination.

The porous granular material may be selected from those having an average pore size in a range from 0.1 to 1000 nm, depending on the type and the like of an impurity contained in the raw material liquid. Note that the average pore size can be measured by mercury intrusion porosimetry or inverse size-exclusion chromatography.

The porous granular material may be selected from those having an average particle size in a range from 1 to 1000 µm depending on the type and the like of an impurity contained in the raw material liquid. The average particle size can be measured by a laser diffraction/scattering method.

In one embodiment, in a case where the minute useful substance contained in the raw material liquid to be treated by the apparatus 1 is a biological substance such as an extracellular vesicle, an antibody, a virus, a protein, an enzyme, or a nucleic acid, the carrier column 21 may include an antibody column. The "antibody column" refers to a column filled with a carrier on which an antibody capable of binding to an antigen (impurity) is immobilized. In a case where the raw material liquid contains the above-described biological substance, an antibody column filled with a carrier on which an antibody capable of binding to (adsorbing) an impurity other than the biological substance is immobilized can be employed. Note that a plurality of types of impurities are often contained in the raw material liquid containing the above-described biomolecule as the minute useful substance, and thus a plurality of antibody columns corresponding to the types of impurities may be used.

Alternatively, instead of the carrier column 21, a module (column module) prepared by combining a plurality of carrier columns may be employed. In a case where a column module is used, the types of adsorbents contained in the respective carrier columns may be the same or different from each other. Inner diameters and lengths of the carrier columns may be the same or different from each other.

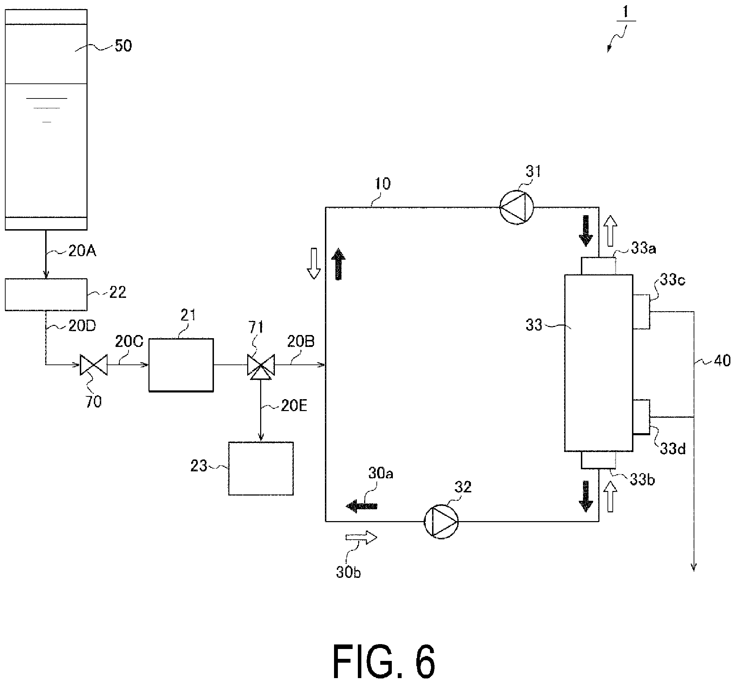

In the apparatus 1 in FIG. 6, a filtration member 22 that removes an impurity from the raw material liquid supplied from the raw material storage unit 50 is interposed in the second flow path. In the apparatus 1 in FIG. 6, the raw material liquid is treated by the filtration member 22 before the liquid is supplied to the carrier column 21. In the apparatus 1 in FIG. 6, the second flow path 20 is divided into the flow path 20A connecting the raw material storage unit 50 and the filtration member 22, a flow path 20D that is a flow path for supplying the liquid treated by the filtration member 22 (hereinafter, referred to as a "pretreated liquid") toward the carrier column 21 and is connected to the on-off valve 70, the flow path 20C connecting the on-off valve 70 and the carrier column 21, and the flow path 20B through which the roughly purified liquid is supplied to the first flow path 10. With the on-off valve 70 opened, the pretreated liquid is supplied to the carrier column 21 via the flow path 20C. According to this configuration, the pretreated liquid pretreated by the filtration member 22 can be supplied to the carrier column 21. Note that although the apparatus 1 in FIG. 6 is configured to include the filtration member 22 and the carrier column 21, it may be configured to include only the filtration member 22.

### Filtration member

The filtration member 22 is a member for filtering the raw material liquid. In a case where the minute useful substance contained in the raw material liquid contains a minute biomolecule such as an extracellular vesicle, the filtration member 22 is preferably a filtration filter including a microfiltration membrane (microfiltration membrane module). From the viewpoint of efficiently filtering and removing an impurity such as a biomolecule larger than the extracellular vesicle of interest, such as a cell in the raw material liquid, the average pore size of the microfiltration membrane (microfiltration membrane module) is preferably from 0.10 to 0.50 µm, and more preferably from 0.15 to 0.30 µm. As the material of the microfiltration membrane, polyether sulfone, polyvinylidene fluoride, or the like is preferable. As the microfiltration membrane, a commercially available product including these materials can be used.

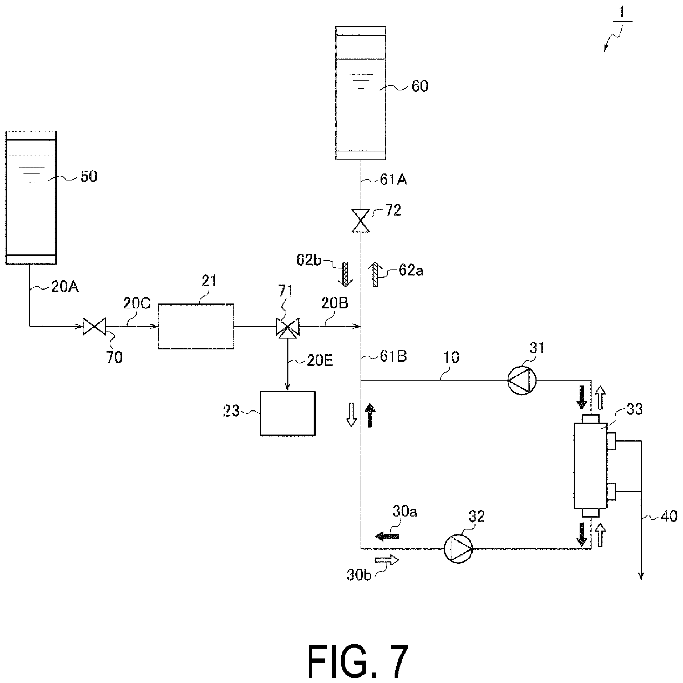

The apparatus 1 in FIG. 7 includes a first tank 60 that stores the raw material liquid (roughly purified liquid) from which an impurity has been removed and the liquid in the first flow path 10. The first tank 60 is connected to a flow path 61A, and the flow path 61A is connected to a flow path 61B via an on-off valve 72. In the apparatus 1 in FIG. 7, the roughly purified liquid treated by the carrier column 21 is fed to the flow path 61B via the flow path 20B, and then flows in an inflow direction 62a to be supplied to the first tank 60. The roughly purified liquid stored in the first tank 60 is supplied to the flow path 61A, then flows in an inflow direction 62b, is fed to the flow path 61B, and is sent to the first flow path 10. The direction in which the liquid flows is indicated by arrows in the drawings. In the apparatus 1 in FIG. 7, of the flow path 61A and the flow path 61B, the flow path flowing in the inflow direction 62b is referred to as a "third flow path 62b".

### First tank

In the apparatus 1 according to an embodiment of the present disclosure, the first tank 60 is a tank that stores the roughly purified liquid (or the pretreated liquid) and the liquid in the first flow path 10. In one embodiment, the first tank 60 may be formed of an infusion pack for easy batch-by-batch removal. Hereinafter, the liquid stored in the first tank 60 will be referred to as a "first stored liquid".

The first tank 60 preferably has transparency in such a manner that a liquid level position of the first stored liquid can be observed. In addition, from the viewpoint of preventing the stored liquid from adhering to and remaining on the inner wall surface of the tank, the first tank 60 is preferably formed of a material having water repellency. Specifically, the first tank 60 is preferably made of a material containing polyacrylonitrile; poly(meth)acrylate such as poly(meth)acrylic acid ester; polycarbonate; a fluorine resin; or the like.

A flow path in a case of treating the raw material liquid in the apparatus 1 in FIG. 7 will be specifically described.

The raw material liquid in the raw material storage unit 50 is supplied to the carrier column 21 through the flow paths 20A and 20C by opening the on-off valve 70. After an impurity is removed by the carrier column 21, when the three-way valve 71 is opened to the flow path 20B side, the roughly purified liquid is supplied from the flow path 20B to the flow path 61B. At this time, if there is no liquid in the first flow path 10, the roughly purified liquid supplied to the flow path 61B flows in the inflow direction 62b and is fed to the first flow path 10. On the other hand, in a case where the purifying and concentrating treatment is performed in the first flow path 10, the roughly purified liquid flows in the inflow direction 62a and is fed toward the flow path 61A. When the on-off valve 72 is opened, the roughly purified liquid is stored in the first tank 60. When the on-off valve 72 is opened, the roughly purified liquid in the first tank 60 can be supplied to the first flow path 10 via the third flow path 62b.

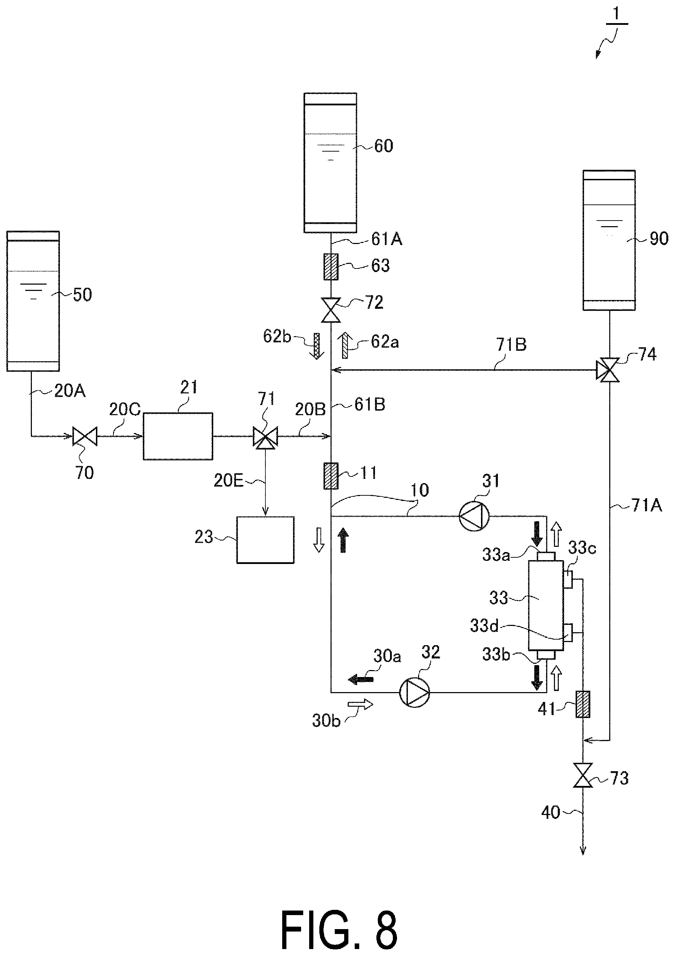

The apparatus 1 in FIG. 8 has the configuration illustrated in FIG. 7 and further includes a second tank 90 that stores a diluent. To the second tank 90, a fourth flow path 71A for supplying the diluent in the tank to the hollow fiber membrane module 33 and a fifth flow path 71B for supplying the diluent to the flow path 61B are respectively connected to a three-way valve 74. In addition, in the apparatus 1 in FIG. 8, the liquid level position monitoring device 11 is interposed in the flow path 61B forming the third flow path 62b. Further, an integrating flowmeter 63 may be interposed in the flow path 61A connected to the first tank 60. In one embodiment, from the viewpoint of making the apparatus 1 more compact and simpler in design, it is particularly preferable that a liquid volume of the first tank 60 be controlled by a flow rate measurement value determined by a clamp-on flowmeter. That is, the integrating flowmeter 63 is particularly preferably a clamp-on integrating flowmeter.

In the apparatus 1 in FIG. 8, the fourth flow path 71A is connected to the flow path 40 connected to the third liquid inlets/outlets 33c and 33d of the hollow fiber membrane module 33. In the apparatus 1 in FIG. 8, the flow path 40 includes an on-off valve 73 and a flowmeter 41. In a case where the diluent is supplied to the fourth flow path 71A, the three-way valve 74 is opened to the fourth flow path 71A side and the on-off valve 73 is closed. This supplies the diluent to the third liquid inlets/outlets 33c and the 33d of the hollow fiber membrane module 33 via the flow path 40. The amount of the diluent supplied to the hollow fiber membrane module 33 is managed by the flowmeter 41. In contrast, in a case where the diluent is supplied to the fifth flow path 71B, the three-way valve 74 is opened to the fifth flow path 71B side, and the on-off valve 72 is opened. This allows the diluent to flow in the inflow direction 62a and be supplied to the flow path 61B. That is, the second tank 90 and the first tank 60 are connected to each other by the fifth flow path 71B. Note that from the viewpoint of making the apparatus 1 more compact and simpler in design, the flowmeter 41 is preferably a clamp-on flowmeter.

Further, the fifth flow path 71B may be connected to the top portion of the first tank 60. Liquid feeding can be performed by means such as a tube pump, a high-pressure gas, or potential energy (hydraulic head difference).

### Diluent

As the diluent stored in the second tank 90, for example, pure water; filtered water of tap water, ground water, river water, or the like; various aqueous solutions; or the like can be used depending on the purpose of the present disclosure and the type of the minute useful substance. In a case where the minute useful substance is an exosome, physiological saline or a medical or biochemical buffer solution is preferably used. As the buffer solution, for example, a phosphate buffer solution (PBS), a tris-hydrochloric acid buffer solution, a sodium citrate buffer solution, a citrate phosphate buffer solution, an acetate buffer solution, a borate buffer solution, or the like can be used.

Note that depending on the purpose of the present disclosure, the diluent preferably contains no impurity. However, in a case where it is difficult to obtain a diluent containing no impurity, a diluent having a low impurity concentration (for example, a diluent having an impurity concentration of less than 500 ppm) can be used.

As described above, the apparatus 1 in FIG. 8 has the configuration of FIG. 7 and includes the second tank 90. The first control unit can control a timing at which the diluent in the second tank 90 is supplied to the hollow fiber membrane module 33 on the basis of the output from the liquid level position monitoring device 11.

### Liquid level position monitoring device

The apparatus 1 in FIG. 8 includes the liquid level position monitoring device 11. In the apparatus 1 in FIG. 8, the liquid level position monitoring device 11 is interposed in the third flow path 62b. In one embodiment, the liquid level position monitoring device 11 is preferably interposed in the immediate vicinity of a connecting portion of the flow path 61B forming the third flow path 62b with the first flow path 10. In a case where the liquid level position monitoring device 11 is provided, a portion of the flow path in which the liquid level position monitoring device 11 is interposed, which is located below the device, can be included in the volume of the first flow path 10. That is, in the apparatus 1 in FIG. 8, the volume of the first flow path 10 can be formed of a portion forming the circulation path 30 and a portion including the flow path below the liquid level position monitoring device 11. In the apparatus 1 in FIG. 8, the flow path 61B is made of a transparent material. The liquid level position monitoring device 11 includes an optical sensor that emits light toward the flow path 61B and detects transmittance of the light. The optical sensor includes a light-emitting element and a light-receiving element. Light is emitted from the light-emitting element toward an inside of the flow path 61A, and the light-receiving element detects intensity (transmittance) of the transmitted light. The detected transmittance is further sent to the detection unit. In a case where the detection unit detects a change in transmittance, the first control unit can control the three-way valve 74 to supply the diluent in the second tank 90 to the fourth flow path 71A or the fifth flow path 71B. Note that the liquid level position monitoring device 11 may be a fiber sensor.

### First control unit

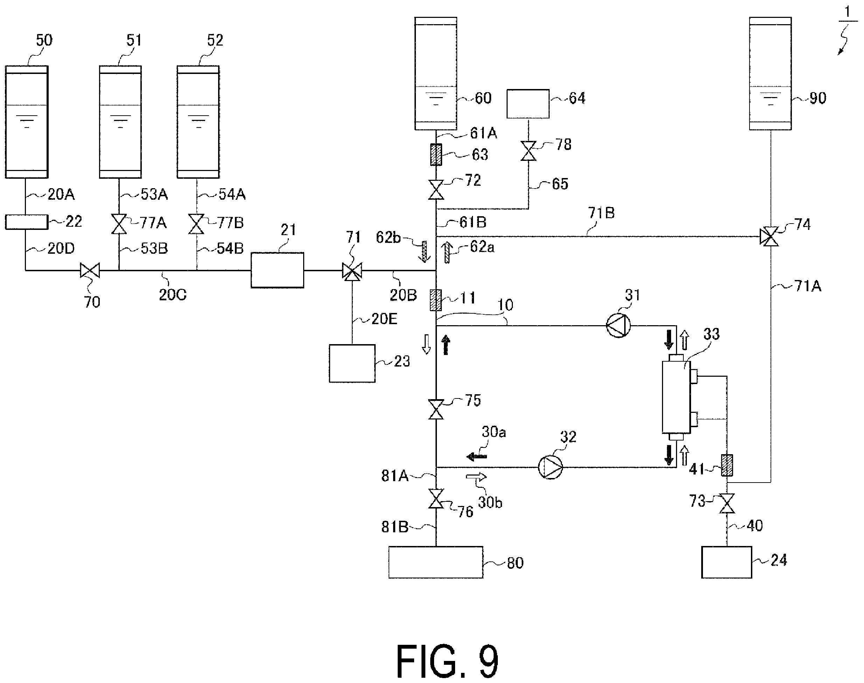

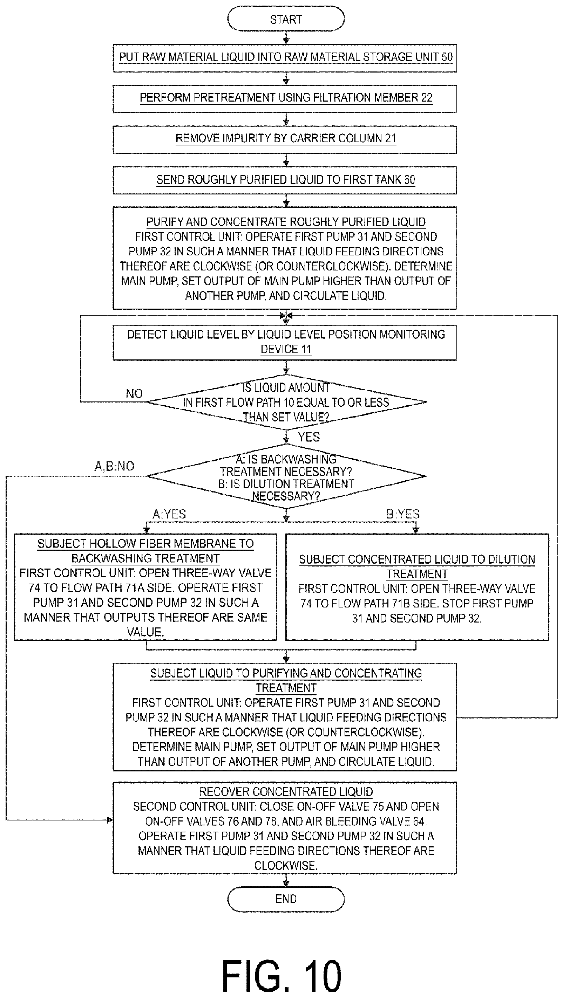

The first control unit of the present disclosure can control the timing at which the diluent in the second tank 90 is supplied to the hollow fiber membrane module 33. An example of determination in diluent flow control of the first control unit is illustrated in the flowchart of FIG. 10. FIG. 10 is a flowchart in a case of performing the purifying and concentrating treatment using the apparatus 1 in FIG. 9.

The relationship between the liquid level position monitoring device 11 and the first control unit will be described more specifically.

In the apparatus 1 in FIG. 8, the first stored liquid in the first tank 60 flows in the inflow direction 62b by the own weight thereof, is supplied to the flow path 61B, and is then fed to the first flow path 10. The first stored liquid is purified and concentrated in the first flow path 10 while circulating through the circulation path 30. As described above, the purifying and concentrating treatment in the first flow path 10 can be performed by operating the one or more pumps interposed in the first flow path 10 to circulate the liquid. This can continuously supply the first stored liquid from the first tank 60 toward the first flow path 10.

When the amount of the first stored liquid in the first tank 60 decreases, a gas in the first tank 60 is discharged toward the third flow path 62b. At this time, when the liquid amount in the first flow path 10 decreases and bubbles reach the liquid level position monitoring device 11 disposed in the flow path 61B, the transmittance of light in the flow path changes. When the liquid level position monitoring device 11 detects a change in transmittance, the first control unit can determine that the liquid amount in the first flow path 10 has decreased, and can open the three-way valve 74 to the fourth flow path 71A side. In addition, the first control unit can operate the one or more pumps in the first flow path 10 (preferably two or more pumps in the first flow path 10) in such a manner that the inside of the hollow fiber membrane module 33 has a negative pressure. In the case of the apparatus 1 in FIG. 8, the first control unit can operate the first pump 31 in such a manner that the liquid in the flow path flows counterclockwise, and can operate the second pump 32 in such a manner that the liquid in the flow path flows clockwise. Further, it is preferable to operate the first pump 31 and the second pump 32 in such a manner that pump outputs of the first pump 31 and the second pump 32 have the same value. This operation makes the inside of the hollow fiber membrane module 33 have a negative pressure. When the inside of the hollow fiber membrane module 33 has a negative pressure, the diluent in the second tank 90 is supplied into the first flow path 10 from the third liquid inlets/outlets 33c and the 33d of the hollow fiber membrane module 33 via the fourth flow path 71A. At this time, the on-off valve 73 in the flow path 40 is closed. The amount of the diluent supplied to the hollow fiber membrane module 33 can be controlled by the flowmeter 41.

The diluent supplied into the module from the third liquid inlets/outlets 33c and the 33d flows from the outside to the inside of the hollow fiber membrane bundle 35. This allows the diluent to flow from the first liquid inlet/outlet 33a and the second liquid inlet/outlet 33b into the first flow path 10 while recovering the minute useful substance deposited on the membrane surfaces of the hollow fiber membranes, particularly on the membrane surfaces inside the hollow fiber membranes. The diluent supplied into the first flow path 10 further flows in the inflow direction 62a and is fed to the first tank 60 while diluting the liquid. Such treatment of supplying the diluent from the permeated liquid side of the hollow fiber membrane module 33 is referred to as "backwashing treatment". By backwashing treatment, the minute useful substance and the impurity deposited on the surfaces of the hollow fiber membranes are peeled off, and the permeation rate of the liquid is likely to be restored to almost the original value. In addition, the liquid is diluted with the diluent to uniformly disperse the impurities and enhance the removal efficiency. As a result, the efficiency of purifying and concentrating the liquid can be further enhanced.

The first control unit can also supply the diluent in the second tank 90 to the first tank 60 via the fifth flow path 71B. For example, in a case where the liquid in the first flow path 10 is diluted without performing the backwashing treatment, the first control unit can open the three-way valve 74 to the fifth flow path 71B side to supply the diluent toward the flow path 61B. Such treatment is referred to as "dilution treatment". In a case of performing the dilution treatment, the first control unit can open the three-way valve 74 to the fifth flow path 71B side in a case where it is determined that the liquid amount in the first flow path 10 has decreased on the basis of the information of the liquid level position monitoring device 11. Furthermore, the first control unit can stop the one or more pumps in the first flow path 10. This allows the diluent supplied to the flow path 61B to be supplied into the first flow path 10, which can dilute the liquid in the flow path. The diluent supplied into the first flow path 10 can flow in the inflow direction 62a and be stored in the first tank 60 while diluting the liquid in the first flow path 10. Note that the amount of the liquid stored in the first tank 60 by the dilution treatment can also be managed by the integrating flowmeter 63. Note that as described above, the integrating flowmeter 63 is particularly preferably a clamp-on integrating flowmeter from the viewpoint of making the apparatus 1 more compact and simpler in design.

In the apparatus 1 in FIG. 8, all the flow paths from the first flow path to the fifth flow path can be configured to be attachable and detachable. For example, all of the first flow path 10, the second flow path 20 (the flow paths 20A, 20B, 20C, and 20E), the third flow path 62b (the flow paths 61A and 61B), the fourth flow path 71A, and the fifth flow path 71B may be formed of resin tubes, and the resin tubes may be connected to the respective elements. When all of the first flow path to the fifth flow path are configured to be attachable to and detachable from other elements as described above, all of the first flow path to the fifth flow path can be detached from other elements and discarded after the purifying and concentrating treatment of the liquid containing the minute useful substance is performed. According to this configuration, the first flow path to the fifth flow path can be discarded and replaced with new flow paths for each batch, and thus foreign matter is less likely to be mixed. In addition, the apparatus 1 can be made to have a simpler configuration.

The sensor portions of the flowmeter and the pressure gauge are preferably in contact with the liquid containing the minute useful substance through the tube (that is, are not in direct contact with the liquid) from the viewpoint of easy attachment and detachment. As the flowmeter, for example, a clamp-on integrating flowmeter or the like can be preferably used.

In the purifying and concentrating apparatus according to the first embodiment of the present disclosure, one or more elements including the first flow path, the second flow path, and the hollow fiber membrane module can be disposed in a housing. From the viewpoint of making the apparatus into which less foreign matter is mixed, it is also possible to adopt a configuration in which all the elements are disposed in the housing.

In addition, a management unit that manages the degree of cleanliness in the housing may be provided. This configuration can keep the inside of the purifying and concentrating apparatus of the present disclosure clean, and makes it easy to prevent contamination of the liquid containing the minute useful substance. The management unit may include a fan filter device for supplying filtered air into the housing and a UV lamp. The combination of these can further enhance the degree of cleanliness in the housing.

### Fan filter device

The fan filter device has a configuration capable of supplying filtered air into the housing. The fan filter device may be a filter unit with a fan incorporating a pre-filter. As the pre-filter, a HEPA filter, an ULPA filter, a chemical filter, a fiber activated carbon filter, or the like can be employed depending on the degree of cleanliness in the housing.

### UV lamp

The UV lamp is not particularly limited as long as it can emit UV (ultraviolet ray) to sterilize the inside of the housing. In addition, the number of the UV lamps disposed in the housing is not particularly limited, and two or more UV lamps may be disposed depending on the degree of cleanliness in the housing.

### Degree of cleanliness

In a case where the purifying and concentrating apparatus according to an embodiment of the present disclosure has a configuration in which each element is disposed in the housing, the degree of cleanliness in the housing can be improved by the above-described management unit. In one embodiment, the degree of cleanliness in the housing may be managed to be class 8 or less, or class 5 or less in the cleanliness class defined by ISO 14644-1:2015.

In addition, the purifying and concentrating apparatus 1 according to an embodiment of the present disclosure can be made to have a compact size, and thus can be carried into a facility such as a clean booth or a clean room in which the degree of cleanliness is managed, which makes the operation easier.

### Minute useful substance

The minute useful substance is preferably selected from the group consisting of an extracellular vesicle, an antibody, a virus, a protein, an enzyme, and a nucleic acid.

### Cartridge

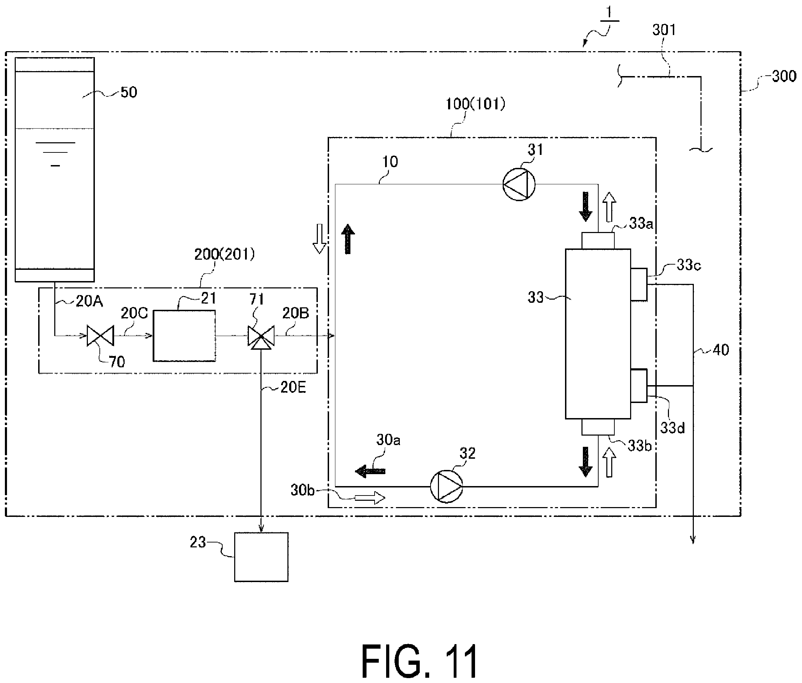

In the purifying and concentrating apparatus 1, each element of the apparatus can be configured as a removable cartridge. According to this configuration, each element can be easily replaced only by replacing the cartridge. Hereinafter, a specific example thereof will be described with reference to FIGS. 11 to 13. In FIGS. 11 to 13, the purifying and concentrating apparatus 1 includes a frame 301 in a housing 300.

### Membrane treatment cartridge

In the purifying and concentrating apparatus 1 in FIG. 11, the hollow fiber membrane module 33 and the first flow path 10 are accommodated in a case 101 of a membrane treatment cartridge 100 that is attachable to and detachable from the frame 301. That is, specifically, in the membrane treatment cartridge 100, the hollow fiber membrane module 33 and the first flow path 10 are accommodated in the case 101, and the case 101 is attachable to and detachable from the frame 301 of the purifying and concentrating apparatus 1 with an attachment member (not illustrated) interposed therebetween. According to this configuration, the hollow fiber membrane module 33 and the first flow path 10 can be easily replaced only by replacing the membrane treatment cartridge 100.

In the purifying and concentrating apparatus 1, at least a part of the first flow path 10 is formed of a resin tube. The first pump 31 and the second pump 32 each are a tube pump capable of locking a part of the first flow path 10, and the tube pump is attached to the frame 301. The tube pump is a device that causes the liquid in a tube constituting a part of the first flow path 10 to flow and includes a roller that presses the tube to cause the liquid in the tube to flow in one direction. In this tube pump, a guide frame is provided to surround the roller, and the tube which is a part of the first flow path 10 is hooked on the roller along the guide frame.

When the membrane treatment cartridge 100 is attached to the frame 301, the third liquid inlets/outlets 33c and 33d of the hollow fiber membrane module 33 are connected to the flow path 40 connected to the waste liquid tank with a connection member interposed therebetween as necessary.

It is preferable that the purifying and concentrating apparatus 1 have a cartridge installation portion in which the membrane treatment cartridge 100 can be disposed on the front side of the apparatus. When the cartridge installation portion is provided on the front side of the apparatus, the cartridge can be replaced more easily.

As a modified example of the above configuration, the first pump 31 and the second pump 32 may be accommodated in the membrane treatment cartridge 100. In this case, the hollow fiber membrane module 33, the first flow path 10, the first pump 31, and the second pump 32 are accommodated in the case 101 of the membrane treatment cartridge 100 that is attachable and detachable. According to this configuration, the hollow fiber membrane module 33, the first flow path 10, the first pump 31, and the second pump 32 can be easily replaced only by replacing the membrane treatment cartridge 100. In this case, the first pump 31 and the second pump 32 that have been used can be reused after being subjected to treatment such as cleaning.

### Pretreatment cartridge

In the purifying and concentrating apparatus 1 in FIG. 11, the second flow path 20 and the carrier column 21 are accommodated in a pretreatment cartridge 200 that is attachable to and detachable from the frame 301 together with the open valve 70 and the three-way valve 71 provided in the second flow path 20. That is, specifically, in the pretreatment cartridge 200, the second flow path 20 (the open valve 70 and the three-way valve 71) and the carrier column 21 are accommodated in a case 201, and the case 201 is attachable to and detachable from the frame 301 of the purifying and concentrating apparatus 1 with an attachment member (not illustrated) interposed therebetween. According to this configuration, the second flow path 20 and the carrier column 21 can be easily replaced only by replacing the pretreatment cartridge 200. When the pretreatment cartridge 200 is attached to the frame 301, the second flow path 20 is connected to the raw material storage unit 50 that stores the raw material liquid of the liquid with a connection member interposed therebetween as necessary.

Connection between the pretreatment cartridge 200 and the membrane treatment cartridge 100 can be performed by connecting the first flow path 10 included in the membrane treatment cartridge 100 and the second flow path 20 included in the pretreatment cartridge 200 with a connection member interposed therebetween as necessary. The connection member may be provided in the membrane treatment cartridge 100 and/or the pretreatment cartridge 200, or may be provided in the frame 301.

The purifying and concentrating apparatus 1 preferably has a cartridge installation portion in which the pretreatment cartridge 200 can be disposed on the front side of the apparatus. When the cartridge installation portion is provided on the front side of the apparatus, the cartridge can be replaced more easily.

### Pretreatment and membrane treatment cartridge 400A

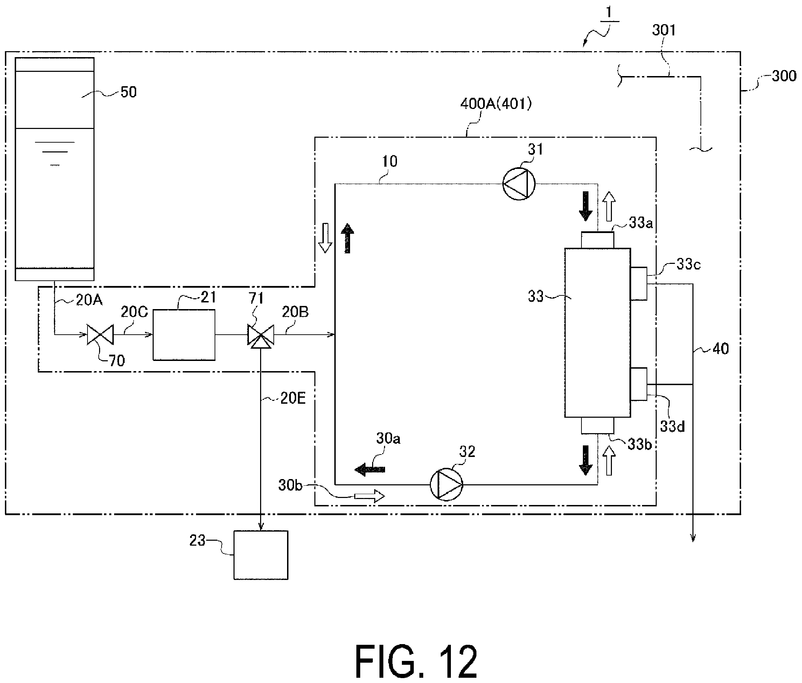

In the purifying and concentrating apparatus 1 in FIG. 12, the hollow fiber membrane module 33, the first flow path 10, the second flow path 20, and the carrier column 21 are accommodated in a case 401 of a pretreatment and membrane treatment cartridge 400A which is attachable to and detachable from the frame 301, together with the open valve 70 and the three-way valve 71 provided in the second flow path 20. That is, specifically, in the pretreatment and membrane treatment cartridge 400A, the hollow fiber membrane module 33, the first flow path 10, the second flow path 20 (the open valve 70 and the three-way valve 71), and the carrier column 21 are accommodated in the case 401, and the case 401 is attachable to and detachable from the frame 301 of the purifying and concentrating apparatus 1 with an attachment member (not illustrated) interposed therebetween. According to this configuration, it is possible to omit a process of connecting the membrane treatment cartridge 100 and the pretreatment cartridge 200 at the time of attachment to the frame 301. In addition, the hollow fiber membrane module 33, the first flow path 10, the second flow path 20, and the carrier column 21 can be easily replaced at a time only by replacing one cartridge. The contents described in the sections of the membrane treatment cartridge 100 and the pretreatment cartridge 200 also apply here.

### Pretreatment and membrane treatment cartridge 400B

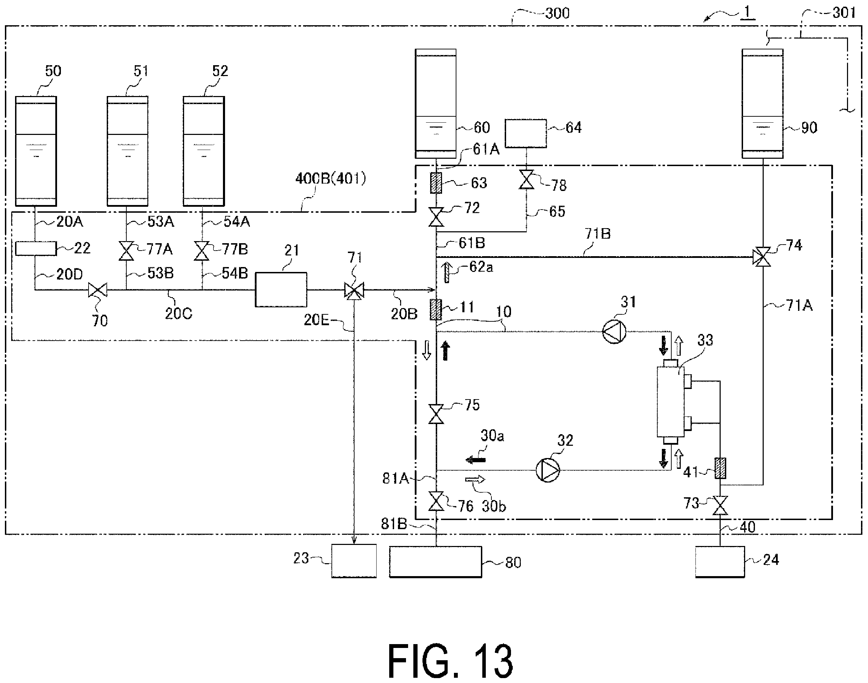

In the purifying and concentrating apparatus 1 in FIG. 13, the hollow fiber membrane module 33, the carrier column 21, the filtration member 22, the first flow path 10, the second flow path 20, the third flow paths 61A and 61B, the fourth flow path 71A, the fifth flow path 71B, the flow paths 40, 53A, 53B, 54A, 54B, 81A, and 81B, an air bleeding line 65, the on-off valves 72, 75, 76, 77A, 77B, and 78, and the three-way valves 71 and 74 are accommodated in a case 401 of a pretreatment and membrane treatment cartridge 400B which is attachable to and detachable from the frame 301. According to this configuration, the respective elements to be replaced for each batch in the purifying and concentrating apparatus 1 can be easily replaced at a time only by replacing one cartridge.

In the purifying and concentrating apparatus 1 in FIG. 13, at least a part of the first flow path 10 is formed of a resin tube similarly to that described above, and the first pump 31 and the second pump 32 each are a tube pump. As described above, the tube pump includes a roller and a guide frame, and is used by hooking the tube, which is a part of the first flow path 10, on the roller along the guide frame.

In the purifying and concentrating apparatus 1 in FIG. 13, at least a part of each of the third flow paths 61A and 61B and the fourth flow path 71A is similarly formed of a resin tube. The liquid level monitoring device 11, the flowmeter 41, and the integrating flowmeter 63 are attached to the frame 301 and each have a tube installation portion in which a tube can be installed. Each tube constituting a part of the third flow paths 61A and 61B or the fourth flow path 71A is installed in the tube installation portion to be used. It is preferable that at least a part of each of the other flow paths be also formed of a resin tube.

When the pretreatment and membrane treatment cartridge 400 is installed in the frame 301, the third flow path 61A, the fourth flow path 71A, and the flow paths 40 and 81B are connected to the first tank 60, the second tank 90, the third tank 80, or the waste liquid tank 24 with connection members interposed therebetween as necessary, respectively. The second flow paths 20 and 20E, and the flow paths 53A and 54A are connected to the raw material storage unit 50, a cleaning liquid storage tank 51, an equilibrating liquid storage tank 52, or the waste liquid tank 23 with connection members interposed therebetween as necessary, respectively. The connection member may be provided in the pretreatment and membrane treatment cartridge 400 or may be provided in the frame 301.

As a modified example of the above-described configuration, one or more other elements selected from the first pump 31, the second pump 32, the liquid level monitoring device 11, and the integrating flowmeter 63 may be accommodated in a membrane treatment and pretreatment cartridge, and the cartridge including the hollow fiber membrane module 33, the flow paths, and the other elements may be attachable to and detachable from the frame 301.

It is preferable that the purifying and concentrating apparatus 1 have a cartridge installation portion in which the pretreatment and membrane treatment cartridges 400A and 400B can be disposed on the front side of the apparatus. When the cartridge installation portion is provided on the front side of the apparatus, the cartridge can be replaced more easily.

Although an embodiment of the apparatus 1 of the present disclosure has been described, the apparatus 1 of the present disclosure is not limited to the combination of the above-described configurations. The apparatus 1 of the present disclosure can be of compact and simple design, which is also one of the features thereof. Accordingly, from the viewpoint of enabling the apparatus 1 of more compact and simpler design, it is possible to omit some or all of the above-described preferred configurations. That is, from the viewpoint of easily enabling the apparatus 1 of more compact and simpler design, it is preferable to adopt a configuration in which the above-described management unit (including the fan filter device and the UV lamp) is omitted.

### Method for Producing Liquid in Which Minute Useful Substance is Purified and Concentrated

Hereinafter, a method for producing a liquid in which the minute useful substance is purified and concentrated using the apparatus 1 according to an embodiment of the present disclosure will be described with reference to FIG. 9. FIG. 9 is a configuration diagram illustrating an example of the overall configuration of the apparatus 1 according to an embodiment of the present disclosure. The production method according to an embodiment of the present disclosure is a method for producing a liquid in which a minute useful substance is purified and concentrated using the apparatus 1, the method including: supplying a raw material liquid containing the minute useful substance to the apparatus 1; and purifying and concentrating the liquid with the hollow fiber membrane module, in which the purifying and concentrating of the liquid is performed while circulating the liquid in the circulation path including the hollow fiber membrane module in a reversible manner.

The production method according to an embodiment of the present disclosure includes supplying a raw material liquid to the apparatus 1. As the raw material liquid, a culture liquid or a dispersion containing a minute useful substance can be employed. The minute useful substance in the raw material liquid is not particularly limited.

In the apparatus 1 in FIG. 9, the raw material liquid is stored in the raw material storage unit 50. Thereafter, the raw material liquid is supplied to the filtration member 22 via the flow path 20A. The raw material liquid is pretreated by the filtration member 22 and then supplied to the flow path 20D. With the on-off valve 70 opened, the pretreated liquid is supplied to the carrier column 21 via the flow path 20C.

In the apparatus 1 in FIG. 9, the tank 51 in which a cleaning liquid is stored (hereinafter, referred to as the "cleaning liquid storage tank 51") and the tank 52 in which an equilibrating liquid is stored (hereinafter, referred to as the "equilibrating liquid storage tank 52") are connected to the flow path 20C. These are used to clean and equilibrate the carrier column 21. That is, the production method according to an embodiment of the present disclosure may include subjecting the carrier column 21 to cleaning and equilibrating treatment.

In a case of subjecting the carrier column 21 to the cleaning and equilibrating treatment, the on-off valve 77A (electromagnetic valve or the like) is first opened to supply the cleaning liquid in the cleaning liquid storage tank 51 to the flow path 20C via the flow paths 53A and 53B. The cleaning liquid supplied to the flow path 20C cleans the carrier column 21 and is then discarded into the waste liquid tank 23 via the flow path 20E. After the cleaning treatment, the on-off valve 77B (electromagnetic valve or the like) is opened to supply the equilibrating liquid in the equilibrating liquid storage tank 52 to the flow path 20C via the flow paths 54A and 54B. The equilibrating liquid supplied to the flow path 20C equilibrates the carrier column 21 and is then discarded into the waste liquid tank 23 via the flow path 20E.

### Cleaning liquid and equilibrating liquid

Examples of the cleaning liquid for cleaning the carrier column 21, which can be employed, include a sodium chloride aqueous solution, hydrochloric acid, citric acid, a sodium hypochlorite aqueous solution, and a sodium hydroxide aqueous solution. From the viewpoint of safety, it is preferable to use a sodium chloride aqueous solution. When the cleaning liquid flows into the carrier column 21, an impurity adhering to the adsorbent in the carrier column 21 is removed. After removal with the cleaning liquid, the carrier column 21 is equilibrated. Examples of the equilibrating liquid that can be employed include physiological saline, a phosphate buffer solution (PBS), a tris-hydrochloric acid buffer solution, a sodium citrate buffer solution, a citrate phosphate buffer solution, an acetate buffer solution, and a borate buffer solution.

Note that the material of the cleaning liquid storage tank 51 that stores the cleaning liquid and the equilibrating liquid storage tank 52 that stores the equilibrating liquid is not particularly limited, and for example, may have the same configuration as that of the first tank 60 and the second tank 90 described above. It is preferable that the cleaning liquid storage tank 51 and the equilibrating liquid storage tank 52 have transparency from the viewpoint of easy visual confirmation of the amount of the stored liquid in the tanks.

Cleaning and equilibration of the carrier column 21 can be set depending on the amount of the raw material liquid treated by the carrier column 21. In one embodiment, the cleaning and equilibration of the carrier column 21 may be performed in a case where as a treated amount by the carrier column 21 relative to the total mass of the raw material liquid, the raw material liquid of from 5 to 100 times the volume of the carrier column 21 has been treated. For example, in a case of treating 1000 mL of the raw material liquid, the cleaning and equilibration treatment of the carrier column 21 is performed every time from 20 to 150 mL of the raw material liquid is treated by the carrier column 21, and the cleaning and equilibration treatment is preferably performed every time from 50 to 100 mL of the raw material liquid is treated.

### Impurity removal treatment

The pretreated liquid supplied to the carrier column 21 is made into a roughly purified liquid by adsorbing and removing an impurity from the liquid by the adsorbent in the carrier column 21, and then supplied to the flow path 61B via the flow path 20B. In one embodiment, the impurity removal treatment by the carrier column 21 may be reducing the concentration of the impurity in the raw material liquid or the pretreated liquid to 1000 ppm or less, and preferably reducing the concentration to 500 ppm or less.

### Purifying and concentrating treatment

The roughly purified liquid supplied to the flow path 61B can flow in the inflow direction 62b and be stored in the first tank 60. The first stored liquid can be supplied to the first flow path 10 via the third flow path 62b depending on the progress of the purifying and concentrating treatment of the liquid in the first flow path 10. The liquid (first stored liquid) supplied into the first flow path 10 is subjected to the purifying and concentrating treatment while being circulated through the circulation paths 30a and 30b.

The liquid in the first flow path 10 can be circulated through the circulation path 30 by operating the one or more pumps interposed in the first flow path 10. In the apparatus 1 in FIG. 9, in a case where both the first pump 31 and the second pump 32 are tube pumps, to circulate the liquid through the circulation path 30a, the liquid feeding directions of the two pumps are set clockwise with respect to each other, one of the first pump 31 and the second pump 32 is set as a main pump, and the output of the main pump is set higher than the output of the other pump. In one embodiment, the main pump is preferably a pump located on the upstream side of the liquid when the hollow fiber membrane module 33 is set as a starting point. In the case of the apparatus 1 in FIG. 9, in a case where the liquid is circulated through the circulation path 30a, it is preferable to set the output of the first pump 31 higher than the output of the second pump 32. On the other hand, in a case where the liquid is circulated through the circulation path 30b, the output of the second pump 32 is preferably set higher than the output of the first pump 31. The output difference of the pumps at this time is preferably 2 to 15%, and more preferably 4 to 20%. Note that in a case where these pumps are tube pumps, the outputs of the first pump 31 and the second pump 32 can be set by changing a discharge flow rate (mL/min) or the output (rotation speed).

In the production method according to an embodiment of the present disclosure, it is preferable that the liquid in first flow path 10 be alternately circulated through circulation paths 30a and 30b. The timing at which the circulation direction of the liquid is switched may be managed depending on the purifying and concentrating time of the liquid or may be managed depending on the flow rate of the liquid circulated in the circulation path 30. Specifically, the timing can be appropriately set depending on a fouling property of the liquid containing the minute useful substance with respect to the hollow fiber membrane. In one embodiment, in a case where the circulation direction is switched depending on the purifying and concentrating time, the circulation direction is preferably switched every 1 second to 10 minutes, and more preferably every 3 seconds to 5 minutes. In a case where the circulation direction is switched depending on the flow rate of the liquid (integrated flow rate after switching the circulation direction of the liquid), the circulation direction is preferably switched every 5 mL to 300 mL, and more preferably every 10 mL to 200 mL.

The liquid in the first flow path 10 can be purified and concentrated while being circulated alternately through the circulation path 30. The liquid having entered the hollow fiber membrane module 33 is purified and concentrated by alternating tangential flow filtration. The permeated liquid is discarded from the third liquid inlets/outlets 33c and 33d of the hollow fiber membrane module 33 to the waste liquid tank 24 via the flow path 40.

In a case where the liquid viscosity of the liquid containing the minute useful substance during operation is from 10 to 30 mPa·s at a liquid temperature of 20°C and a shear rate of 25 s⁻¹, a large-diameter hollow fiber membrane having an average inner diameter of from 1.0 mm to 1.4 mm can be used.

In one embodiment, the liquid in the first flow path 10 may be circulated in such a manner that a hollow fiber transmembrane pressure in the hollow fiber membrane module 33 is from 0.02 to 0.2 MPa. The pressure is preferably from 0.02 to 0.15 MPa, and more preferably from 0.03 to 0.12 MPa. Note that the hollow fiber transmembrane pressure is a differential pressure between a pressure applied to the inner surface of the hollow fiber membrane and a pressure applied to the outer surface of the hollow fiber membrane.

### Backwashing treatment

The production method according to an embodiment of the present disclosure can include subjecting the liquid to alternating tangential flow filtration in the hollow fiber membrane module 33 while backwashing the hollow fiber membrane to recover the minute useful substance deposited on the membrane surface of the hollow fiber membrane. The backwashing treatment can be performed by supplying the diluent in the second tank 90 to the third liquid inlets/outlets side of the hollow fiber membrane module 33 in a case where the liquid amount in the first flow path 10 is equal to or less than a set value. As a method of setting a value for the liquid amount in the first flow path 10, for example, in a case where the apparatus 1 in FIG. 9 is used, it may be set to the liquid amount when the liquid level position monitoring device 11 detects a change in transmittance. In this case, the first control unit can open the three-way valve 74 to the fourth flow path 71A side, whereby the backwashing treatment can be performed. In addition, the liquid feeding direction of the first pump 31 can be set counterclockwise and the liquid feeding direction of the second pump 32 can be set clockwise in such a manner that the inside of the hollow fiber membrane module 33 has a negative pressure. Further, it is preferable that the first pump 31 and the second pump 32 be operated with the same output value (the output difference between the first pump 31 and the second pump 32 is in a range of from 0 to 4%). At this time, the outputs of the first pump 31 and the second pump 32 can be set. From the viewpoint of energy saving, the outputs of the first pump 31 and the second pump 32 may be 30% or less, or may be 20% or less.

In one embodiment, the backwashing treatment can be appropriately performed even when the liquid amount in the first flow path 10 is equal to or less than a set value. Examples thereof include a case where there is a concern about a decrease in permeation rate from a situation of pressure fluctuation or flow rate fluctuation, a case where a tendency of a decrease in permeation rate can be determined, and a case where a safety operation for preventing occurrence of clogging of the hollow fiber membrane is performed.

In a case where the backwashing treatment is performed, it is preferable to supply the diluent from the hollow fiber membrane module 33 into the first flow path 10 in an amount of from 2 times to 200 times the liquid amount set by the liquid level position monitoring device 11. At this time, an excessive amount of the diluent flows in the inflow direction 62a while diluting the liquid in the first flow path 10 and is stored in the first tank 60. It is preferable that the amount of the diluent be managed by the flowmeter 41. In one embodiment, the amount of the diluent is more preferably from 2 times to 20 times the amount of the liquid. Such backwashing treatment allows the concentrated liquid to be diluted while recovering the minute useful substance deposited on the membrane surface of the hollow fiber membrane, which is preferable.

Furthermore, the purifying and concentrating efficiency may be enhanced by maintaining the amount of the diluent at from 2 to 10 times the amount of the liquid in the initial stage of the purifying and concentrating treatment (e.g., a stage of the purifying and concentrating treatment of the roughly purified liquid) and increasing the amount of the diluent to from 2 to 15 times the amount of the diluent supplied in the initial stage in the intermediate stage or later stages of the purifying and concentrating treatment (e.g., a stage where the liquid subjected to the dilution treatment once or more is subjected to the purifying and concentrating treatment again).

Note that before performing the backwashing treatment, to prevent the permeated liquid remaining in the hollow fiber membrane module from being mixed with the backwashing liquid and flowing into the concentrated liquid side, an operation of performing a flushing treatment with a cleaning liquid (diluent) in advance and replacing the permeated liquid remaining in the hollow fiber membrane module with the cleaning liquid (diluent) may be optionally performed.

At this time, an amount of the cleaning liquid (diluent) to be used is preferably 1 to 8 times, and more preferably 2 to 6 times the volume of the permeated liquid remaining in the hollow fiber membrane module.

**In** a case of concentrating and purifying a certain type of extracellular vesicles in a certain type of culture supernatant, performing the flushing operation before the backwashing may reduce a residual amount of impurity proteins to half or less as compared with not performing the flushing operation.

In a case where the hollow fiber membrane module 33 is of an internal pressure type, an operation of replacing the permeated liquid remaining in the hollow fiber membrane module with a cleaning liquid (diluent) can be performed by using one of the third liquid inlets/outlets 33c and 33d in FIG. 2 as an inlet and the other as an outlet.

The hollow fiber membrane has liquid passing resistance. Thus, when the diluent is supplied from one of the third liquid inlets/outlets 33c and 33d, the permeated liquid remaining in the hollow fiber membrane module 33 can be replaced with the diluent in a state in which leakage to the membrane side is small.

For example, the flow of the liquid through the first liquid inlet/outlet 33a and the second liquid inlet/outlet 33b is stopped by stopping the liquid flow through the first pump 31 and the second pump 32. In this state, when the diluent is supplied from one of the third liquid inlets/outlets 33c and 33d using a pump or the like (not illustrated), the diluent is discharged from the other of the third liquid inlets/outlets 33c and 33d. This makes it possible to replace the permeated liquid remaining in the hollow fiber membrane module 33 with the diluent.

Further, in a state where the liquid passing through the first pump 31 and the second pump 32 is stopped, a valve (not illustrated) is closed to stop the flow of the liquid through the first liquid inlet/outlet 33a and the second liquid inlet/outlet 33b. In this state, when the diluent is supplied from one of the third liquid inlets/outlets 33c and 33d using a pump or the like (not illustrated), the diluent is discharged from the other of the third liquid inlets/outlets 33c and 33d. This makes it possible to replace the permeated liquid remaining in the hollow fiber membrane module 33 with the diluent.

The timing of the flushing treatment is controlled by the first control unit. That is, on the basis of the output of the liquid level position monitoring device, the first control unit can supply the diluent in the second tank 90 from any one of the at least two third liquid inlets/outlets 33a and 33b of the hollow fiber membrane module 33 in a state where the liquid passing through the first pump 31 and the second pump 32 is stopped and the flow of the liquid through the first liquid inlet/outlet 33c and the second liquid inlet/outlet 33d is stopped by closing a valve (not illustrated). At this time, the on-off valve 73 provided in the flow path 40 connected to the waste liquid tank is opened.

Thereafter, the valve is opened to make a state in which a liquid can flow through the first liquid inlet/outlet 33a and the second liquid inlet/outlet 33b, and the diluent in the second tank 90 is supplied from any one of the at least two third liquid inlets/outlets 33c and 33d of the hollow fiber membrane module 33 using the first and second pumps 31 and 32 or another pump or the like (not illustrated), whereby backwashing can be performed. At this time, the on-off valve 73 provided in the flow path 40 connected to the waste liquid tank is closed.

### Dilution treatment

In the production method according to an embodiment of the present disclosure, further purifying and concentrating treatment can be performed while diluting the concentrated liquid that has been purified and concentrated. In one embodiment, the dilution ratio of the concentrated liquid is preferably from 2 to 200 times, and more preferably from 2 to 20 times. The dilution treatment can be performed in a case where the amount of the liquid (concentrated liquid) in the first flow path 10 is equal to or less than a set value. In the case of using the apparatus 1 in FIG. 9, the value can be set to the liquid amount when the liquid level position monitoring device 11 detects a change in transmittance, as in the case of the above-described backwashing treatment. In a case of performing the dilution treatment, the first control unit can also open the three-way valve 74 to the fifth flow path 71B side. It is preferable that the first control unit stop the first pump 31 and the second pump 32 when the dilution treatment is performed. In one embodiment, the purifying and concentrating treatment can be performed in parallel with the dilution treatment. When the purifying and concentrating treatment is performed in parallel with the dilution treatment, the fifth flow path 71B is preferably connected to the top portion of the first tank 60. The diluent can be fed to the first tank 60 by means such as a tube pump, high-pressure gas, or potential energy (hydraulic head difference). When the first pump 31 and the second pump 32 are stopped during the dilution treatment, the diluent supplied to the fifth flow path 71B is supplied from the flow path 61B into the first flow path 10, and then flows in the inflow direction 62a to be supplied to the first tank 60.

In the production method according to an embodiment of the present disclosure, the removal of an impurity from the raw material liquid by the carrier column 21 and the purifying and concentrating of the liquid by the hollow fiber membrane module 33 are preferably performed in parallel. In this case, it is possible to more efficiently produce a liquid in which the minute useful substance is purified and concentrated. In the apparatus 1 in FIG. 9, the roughly purified liquid from which the impurity has been removed by the carrier column 21 flows in the inflow direction 62a and is stored in the first tank 60. The first stored liquid in the first tank 60 can be supplied to the first flow path 10 via the third flow path 62b in the progress of the purifying and concentrating treatment.

### Recovery process

The production method according to an embodiment of the present disclosure may include entirely recovering the concentrated liquid in the first flow path 10. The concentrated liquid in the first flow path 10 is entirely recovered into the third tank 80 via the flow paths 81A and 81B by closing the on-off valve 75 at a freely-selected timing while opening the on-off valve 76 and supplying clean gas from the air bleeding portion using the first pump 31 and the second pump 32. In the apparatus 1 in FIG. 9, the second control unit opens the on-off valve 76, and further opens an air bleeding valve 64 and an on-off valve 78. The air bleeding valve 64 is connected to the flow path 61B via the air bleeding line 65. The on-off valve 78 is interposed in the air bleeding line 65. Thereafter, the second control unit closes the on-off valve 75 at a freely-selected timing, whereby the concentrated liquid can be recovered.

Further, the first pump 31 and the second pump 32 can be operated in such a manner that the concentrated liquid in the first flow path 10 circulates through the circulation path 30a. That is, the liquid feeding directions of the first pump 31 and the second pump 32 can be operated in the same clockwise direction. At this time, the outputs of the first pump 31 and the second pump 32 can be set. In one embodiment, from the viewpoint of energy saving, the outputs of the first pump 31 and the second pump 32 are preferably 50% or less, and more preferably 30% or less. In addition, the output difference between the first pump 31 and the second pump 32 is preferably about the same (for example, in a range of from 0 to 4%). The operation of recovering the concentrated liquid in the first flow path 10 can be manually operated by a person.

### Operation of cleaning out minute useful substance deposited on hollow fiber membrane surface immediately before recovery treatment

The production method according to an embodiment of the present disclosure may include an operation of cleaning out the minute useful substance deposited on the hollow fiber membrane surface immediately before the recovery treatment.

As an example, in a case of concentrating and purifying a certain type of extracellular vesicles in a certain type of culture supernatant, the extracellular vesicles which are relatively firmly deposited on the surface of the hollow fiber membrane are not dispersed in the concentrated liquid, and thus the recovery rate may be low in the recovery treatment.

In particular, in a case where the amount of the concentrated liquid in the first flow path 10 is small, when extracellular vesicles relatively firmly deposited on the surface of the hollow fiber membrane cannot be recovered, the purifying and concentrating efficiency is reduced. Thus, an operation process of re-dispersing the extracellular vesicles deposited on the surface of the hollow fiber membrane into the concentrated liquid with a small amount of the backwashing liquid (diluent) can be performed at a timing immediately before the recovery treatment. At this time, it is preferable that the flushing operation be sufficiently performed to reduce the amount of the backwashing liquid (diluent) to be used, and the volume of the backwashing liquid (diluent) to be used is preferably, as an example, from half to twice the volume of the first flow path.

The liquid flow rate at this time can be made equal to the liquid flow rate in the purifying and concentrating process.

### Example 1

Here, a method for further improving the recovery rate of the minute useful substance by coating the surface of the hollow fiber membrane with a biomolecule was examined.

As the raw material liquid, 790 mL of a culture supernatant of mesenchymal stem cells having a protein concentration of 0.49 mg/mL and an exosome concentration of 166.4 pg/mL was used.

The concentration of exosomes was an amount corresponding to a CD9/CD63 fusion standard protein used for preparing the calibration curve when quantitative analysis was performed by a CD9/CD63 ELISA method (EXH0102EL, Cosmo Bio Co., Ltd.).

To 790 mL of the culture supernatant, 79 mL of a carrier equilibrated with 50 mM Tris-HCl (pH 7.2) (a porous granular material in which the insides of pores are positively charged, an adsorbent capable of adsorbing and holding negatively charged unnecessary substances except extracellular vesicles) was added, and mixed by inversion at room temperature for 10 minutes using a rotator. Subsequently, the carrier was removed from the carrier-cell supernatant mixture using a 0.22 µm filtration unit (S2GPU11RE, Merck Millipore) to prepare a carrier-treated culture supernatant.

Next, the carrier-treated culture supernatant was purified and concentrated by filtration in an alternating tangential flow scheme at a filtration pressure of from 30 to 40 kPa. As the filtration membrane, a cellulose acetate hollow fiber membrane module (molecular weight cutoff of 300000, membrane area of 0.062 m²) was used. The filtration membrane having a membrane surface coated with a biomolecule was used. The coating treatment was performed by feeding 1.3 mg/mL of a skim milk solution dissolved in PBS through the filtration membrane at a liquid flow rate of 200 mL/min for 10 minutes. As a result, a coated membrane was produced in which a certain type of biomolecule in skim milk was adsorbed and bonded as a thin layer to the membrane surface. The filter membrane was then rinsed with 250 mL of PBS to remove excess skim milk just deposited on the thin layer.

Here, the bonding state between the filtration membrane and the certain type of biomolecule in the skim milk was strengthen to such an extent that the biomolecule did not fall off under washing with water and operation conditions, and that the filtration performance hardly decreased even in a long-term operation.

The alternating tangential flow filtration was repeated until the liquid amount of the concentrated liquid became 10 mL, and then 90 mL of PBS was supplied to the hollow fiber membrane module to dilute the concentrated liquid. These concentration-filtration, and dilution processes were further repeated twice, followed by alternating tangential flow filtration to produce 11.9 mL of a purified and concentrated liquid (purified extracellular vesicles). The extracellular vesicles in the finally resulting concentrated liquid of exosomes were quantitatively analyzed by the CD9/CD63 ELISA method (EXH0102EL, Cosmo Bio Co., Ltd.). As a result, the concentration was 3746.7 pg/mL corresponding to the CD9/CD63 fusion standard protein used for preparing the calibration curve, and the recovery rate was 33.9%.

### Comparative Example 1

When the same operation was performed using a hollow fiber membrane not coated with skim milk, the recovery rate was 7.4%.

From the results of Example 1 and Comparative Example 1, it has been found that the recovery rate of the minute useful substance is improved by using the hollow fiber membrane coated with a biomolecule (that is, the hollow fiber membrane in which a biomolecule of a type different from the minute useful substance is attached to at least a partial region).

### Example 2

Here, a method for further improving the purity of the minute useful substance in the purified and concentrated liquid prepared was examined.

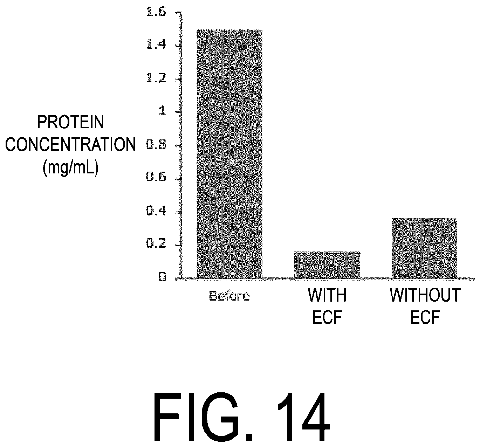

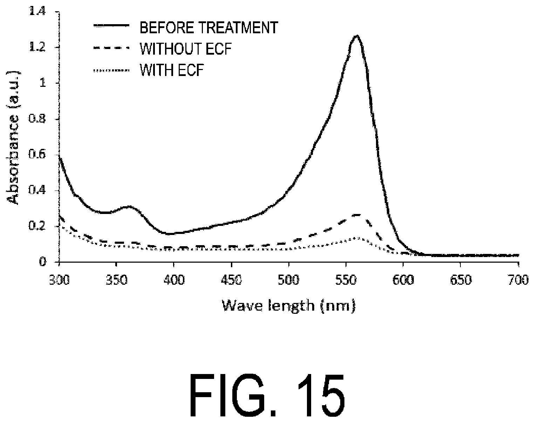

As the raw material liquid, 40 mL of a culture supernatant of amnion-derived mesenchymal stem cells having a protein concentration of 1.49 mg/mL and an exosome concentration of 196 pg/mL was used. The exosomes in the culture supernatant were separated, purified, and concentrated by the alternating tangential flow scheme at a filtration pressure of from 30 to 40 kPa using a purifying and concentrating apparatus equipped with the hollow fiber membrane module 33 made of cellulose acetate (molecular weight cutoff of 300000, membrane area of 0.01 m², permeation-side volume (i.e., extracapillary space (ECS) of 10 mL). After the culture supernatant was concentrated to about 10 mL, 40 mL of the diluent (PBS) was injected from the third liquid inlet/outlet 33c of the hollow fiber membrane module and discharged to the third liquid inlet/outlet 33d. This flushed the impurity in the permeated liquid retaining in the ECS with PBS. Thereafter, 90 mL of the same diluent (PBS) was injected under pressure from the permeation side to the concentration side of the hollow fiber membrane module 33 to perform back pressure washing (backwashing). Separation and purification operations were performed again under the same conditions, and when the culture supernatant reached about 10 mL, similar flushing and backwashing were further performed twice. After backwashing was performed three times, the culture supernatant was concentrated to 5.5 mL. When the residual amount of protein in the resulting purified and concentrated liquid was measured by BCA assay, the protein concentration was 0.16 mg/mL. The results are shown in FIG. 14. Further, an absorbance of the resulting purified and concentrated liquid was measured using an absorption microplate reader Infinite M Nano+ available from Tecan Trading AG. The results are shown in FIG. 15.

### Comparative Example 2

In the same separation and purification operations as in Example 2, the culture supernatant was concentrated to 5.2 mL without flushing the ECS with PBS. When the residual amount of protein in the resulting purified and concentrated liquid was measured by BCA assay, the protein concentration was 0.36 mg/mL. The results are shown in FIG. 14. In addition, an absorbance of the resulting purified and concentrated liquid was measured in the same manner as in Example 2. The results are shown in FIG. 15.

In FIGS. 14 and 15, "BEFORE" represents a sample before the purifying and concentrating treatment, "WITH ECF" represents the purified and concentrated liquid prepared by the method of Example 2, and "WITHOUT ECS" represents the purified and concentrated liquid prepared by the method of Comparative Example 2. As is clear from FIG. 14, according to the method of Example 2, the concentration of protein as an impurity is further reduced, and the purity of exosomes can be further improved. In FIG. 15, the peak near the wavelength of 557 nm is a peak derived from phenol red contained as a pH indicator in the culture liquid and corresponds to an impurity in the purified and concentrated liquid. As shown in FIG. 15, according to the method of Example 2, the concentration of phenol red as an impurity is further reduced, and the purity of exosomes can be further improved.

From these results, it has been found that when the inside of the ECS is washed away (flushed) with a diluent such as PBS or physiological saline before the backwashing process of the hollow fiber membrane module to remove the impurity remaining in the ECS, the concentration of the impurity in the resulting purified and concentrated liquid can be further reduced, and as a result, the purity of the minute useful substance can be further improved. As a non-limiting reason, it is considered that flushing can prevent an impurity in the ECS from re-entering the purified and concentrated liquid during the subsequent backwashing process with the diluent.

### Example 3

As the raw material liquid, 200 mL of a culture supernatant of amnion-derived mesenchymal stem cells having a protein concentration of 1.25 mg/mL and an exosome concentration of 138 pg/mL was used. Exosomes in the culture supernatant were separated, purified, and concentrated by the alternating tangential flow scheme using a purifying and concentrating apparatus equipped with a hollow fiber membrane module made of cellulose acetate (molecular weight cutoff of 300000, membrane area of 0.01 m², ESC of 10 mL). After the culture supernatant was concentrated to about 17 mL, 40 mL of the diluent (PBS) was injected from the third liquid inlet/outlet 33c of the hollow fiber membrane module and discharged to the third liquid inlet/outlet 33d. This flushed the impurity in the permeated liquid retaining in the ECS with PBS. Thereafter, 160 mL of the same diluent (PBS) was injected under pressure from the permeation side to the concentration side of the hollow fiber membrane module 33 to perform back pressure washing (backwashing). Separation and purification operations were performed again under the same conditions, and when the culture supernatant reached about 17 mL, similar flushing and backwashing were further performed twice. The culture supernatant after backwashing was concentrated to 50 mL. Backwashing was performed again to wash out minute useful substances adhering to the surfaces of the hollow fiber membrane into the purified and concentrated liquid, and 50 mL of the purified and concentrated liquid was recovered. The extracellular vesicles in the resulting purified and concentrated liquid were quantitatively analyzed by the CD9/CD63 ELISA method (EXH0102EL, Cosmo Bio Co., Ltd.) to find that the concentration was 486 pg/mL corresponding to the CD9/CD63 fusion standard protein used for preparing the calibration curve, and the recovery rate was 88%. From this result, it has been found that when the diluent is further supplied to the hollow fiber membrane module immediately before recovering the purified and concentrated liquid to wash out the minute useful substance, the recovery rate of the minute useful substance can be increased to 80% or more.

### Reference Signs List

1 Purifying and concentrating apparatus, 10 First flow path, 11 Liquid level position monitoring device, 20 (20A to 20E) Second flow path, 21 Carrier column, 22 Filtration member, 23, 24 Waste liquid tank, 30a, 30b Circulation path (circulation path 30), 31 First pump, 32 Second pump, 33 Hollow fiber membrane module, 41 Flowmeter (clamp-on flowmeter), 50 Raw material storage unit, 51 Cleaning liquid storage tank, 52 Equilibrating liquid storage tank, 60 First tank, 61A, 61B Flow path, 62a, 62b Inflow direction, 63 Integrating flowmeter (clamp-on integrating flowmeter), 64 Air bleeding valve, 65 Air bleeding line, 80 Third tank, 90 Second tank, 100 Membrane treatment cartridge, 200 Pretreatment cartridge, 300 Housing, 301 Frame, 400A, 400B Pretreatment and membrane treatment cartridge, 101, 201, 401 Case

## Claims

1. A purifying and concentrating apparatus for purifying and concentrating a liquid containing a minute useful substance, the purifying and concentrating apparatus comprising:
a hollow fiber membrane module having a plurality of hollow fiber membranes accommodated in a container, the container having a first liquid inlet/outlet and a second liquid inlet/outlet for supplying a liquid containing a minute useful substance to the plurality of hollow fiber membranes, and a third liquid inlet/outlet for discharging the liquid having permeated through the plurality of hollow fiber membranes;
a first flow path connected to the first liquid inlet/outlet and the second liquid inlet/outlet and forming a circulation path including the hollow fiber membrane module;
a second flow path through which the liquid is supplied to the first flow path; and
one or more pumps interposed in the first flow path and configured to circulate the liquid in a reversible manner.

2. The purifying and concentrating apparatus according to claim 1, wherein the one or more pumps each are a tube pump.

3. The purifying and concentrating apparatus according to claim 1 or 2, wherein a volume of the first flow path is from 5 to 100 mL.

4. The purifying and concentrating apparatus according to claim 1 or 2, wherein the one or more pumps include at least a first pump disposed on the first liquid inlet/outlet side and a second pump disposed on the second liquid inlet/outlet side in the first flow path.

5. The purifying and concentrating apparatus according to claim 1 or 2, wherein each of the plurality of hollow fiber membranes has an average inner diameter of from 0.2 to 1.4 mm and a molecular weight cutoff of from 10000 to 1000000.

6. The purifying and concentrating apparatus according to claim 1 or 2, wherein the first flow path is configured to be attachable and detachable.

7. The purifying and concentrating apparatus according to claim 1 or 2, wherein the second flow path is connected to a raw material storage unit that stores a raw material liquid of the liquid containing the minute useful substance.

8. The purifying and concentrating apparatus according to claim 7, wherein a carrier column that removes an impurity from the liquid supplied from the raw material storage unit is interposed in the second flow path.

9. The purifying and concentrating apparatus according to claim 7, wherein a filtration member that removes an impurity from the liquid supplied from the raw material storage unit is interposed in the second flow path.

10. The purifying and concentrating apparatus according to claim 8, further comprising a first tank that stores the liquid from which an impurity has been removed and the liquid in the first flow path,
wherein
the first tank is connected to the second flow path, and
the first tank is connected to a third flow path through which the liquid in the first tank is supplied toward the first flow path.

11. The purifying and concentrating apparatus according to claim 10, further comprising a second tank that stores a diluent, the second tank being connected to a fourth flow path through which the diluent is supplied to the hollow fiber membrane module.

12. The purifying and concentrating apparatus according to claim 11, wherein the second tank and the first tank are connected to each other by a fifth flow path.

13. The purifying and concentrating apparatus according to claim 12, wherein all of the first flow path to the fifth flow path are configured to be attachable and detachable.

14. The purifying and concentrating apparatus according to claim 11, further comprising:
a liquid level position monitoring device including an optical sensor configured to emit light toward the third flow path and detect a transmittance of the light; and
a first control unit configured to control a timing at which the diluent in the second tank is supplied to the hollow fiber membrane module based on an output of the liquid level position monitoring device.

15. The purifying and concentrating apparatus according to claim 14, wherein the first control unit controls a timing at which the diluent in the second tank is supplied from the third liquid inlet/outlet of the hollow fiber membrane module.

16. The purifying and concentrating apparatus according to claim 1 or 2, further comprising a third tank connected to the first flow path and configured to recover the liquid in the first flow path.

17. The purifying and concentrating apparatus according to claim 1 or 2, wherein one or more elements of the first flow path, the second flow path, and the hollow fiber membrane module are disposed in a housing.

18. The purifying and concentrating apparatus according to claim 17, further comprising a management unit configured to manage a degree of cleanliness in the housing,
wherein
the management unit includes a fan filter device that supplies filtered air.

19. The purifying and concentrating apparatus according to claim 18, wherein the management unit includes a UV lamp.

20. The purifying and concentrating apparatus according to claim 1 or 2, wherein the minute useful substance is selected from the group consisting of an extracellular vesicle, an antibody, a virus, a protein, an enzyme, and a nucleic acid.

21. The purifying and concentrating apparatus according to claim 1 or 2, wherein at least the hollow fiber membrane module and the first flow path are accommodated in a case of an attachable and detachable cartridge.

22. The purifying and concentrating apparatus according to claim 8, wherein at least the second flow path and the carrier column are accommodated in a case of an attachable and detachable cartridge.

23. The purifying and concentrating apparatus according to claim 22, wherein the hollow fiber membrane module and the first flow path are accommodated in the case.

24. The purifying and concentrating apparatus according to claim 15, wherein
the hollow fiber membrane has at least two third liquid inlets/outlets, and
the first control unit supplies the diluent from one of the at least two third liquid inlets/outlets in a state where flow of the liquid through the first liquid inlet/outlet and the second liquid inlet/outlet of the hollow fiber membrane module is stopped, and then supplies the diluent from one of the at least two third liquid inlets/outlets in a state where the flow of the liquid through the first liquid inlet/outlet and the second liquid inlet/outlet of the hollow fiber membrane module is enabled.

25. A method for producing a purified and concentrated liquid containing a minute useful substance using the purifying and concentrating apparatus described in claim 1 or 2, the method comprising:
supplying a raw material liquid of a liquid containing a minute useful substance to the purifying and concentrating apparatus; and
purifying and concentrating the liquid with the hollow fiber membrane module,
wherein
the purifying and concentrating the liquid is performed while circulating the liquid in the circulation path including the hollow fiber membrane module in a reversible manner.

26. The method for producing a purified and concentrated liquid according to claim 25, the method further comprising changing a circulation direction of the liquid in the first flow path depending on a purifying and concentrating time of the liquid or depending on a flow rate of the liquid circulated in the circulation path.

27. The method for producing a purified and concentrated liquid according to claim 25, wherein the liquid is supplied to the hollow fiber membrane module to be purified and concentrated in such a manner that a hollow fiber transmembrane pressure in the hollow fiber membrane module is from 0.02 to 0.2 MPa.

28. The method for producing a purified and concentrated liquid according to claim 27, the method further comprising feeding the liquid in the first flow path toward the hollow fiber membrane module by
setting a liquid feeding direction of a first pump disposed on the first liquid inlet/outlet side, and a liquid feeding direction of a second pump disposed on the second liquid inlet/outlet side to be clockwise or counterclockwise, the first pump and the second pump being among two or more of the one or more pumps interposed in the first flow path, and
setting an output of one of the first pump and the second pump as a main pump to be higher than an output of the other pump.

29. The method for producing a purified and concentrated liquid according to claim 25, wherein the purifying and concentrating the liquid with the hollow fiber membrane module includes subjecting the liquid to alternating tangential flow filtration with the hollow fiber membrane module.

30. The method for producing a purified and concentrated liquid according to claim 25, wherein the purifying and concentrating the liquid with the hollow fiber membrane module includes subjecting the liquid to alternating tangential flow filtration with the hollow fiber membrane module while recovering the minute useful substance deposited on membrane surfaces of the hollow fiber membrane.

31. The method for producing a purified and concentrated liquid according to claim 25, wherein
the purifying and concentrating the liquid with the hollow fiber membrane module includes diluting the concentrated liquid with a diluent by from 2 to 200 times, and then further purifying and concentrating the diluted liquid, and
a timing of supplying a diluent to the concentrated liquid is determined by a liquid level position of the liquid in the first flow path.

32. The method for producing a purified and concentrated liquid according to claim 25, the method further comprising recovering an entire amount of the purified and concentrated liquid in the first flow path.

33. The method for producing a purified and concentrated liquid according to claim 25, wherein the minute useful substance is selected from the group consisting of an extracellular vesicle, an antibody, a virus, a protein, an enzyme, and a nucleic acid.

34. The method for producing a purified and concentrated liquid according to claim 25, wherein the purifying and concentrating the liquid with the hollow fiber membrane module includes subjecting the hollow fiber membrane module to flushing treatment.
